# EUROPEAN PATENT SPECIFICATION

(11) **EP 2 420 221 B1**
(45) Date of publication and mention of the grant of the patent: **28.09.2016**
(21) Application number: 10764536.8
(22) Date of filing: 16.04.2010
(51) Int. Cl.: A61K 8/42, A61K 8/81, A61Q 1/10, A61Q 1/12, A61K 8/02, A61K 8/37, A61K 8/69, A61K 8/41, A61Q 13/00

(54) **Solid powder cosmetic and method for producing the same**
Feststoffpulver Kosmetikum und Verfahren zur Herstellung davon
Produit cosmétique sous forme de poudre solide et procédé pour sa fabrication

(30) Priority: 16.04.2009 JP 2009099938
(43) Date of publication of application: 22.02.2012
(73) Proprietor: Shiseido Company, Ltd., Chuo-ku Tokyo 104-8010 (JP)
(72) Inventor: KANEKO Katsuyuki, Yokohama-shi Kanagawa 224-8558 (JP); SONOYAMA Yuji, Yokohama-shi Kanagawa 224-8558 (JP); KURAHASHI Takuma, Yokohama-shi Kanagawa 224-8558 (JP); KUSABA Kentaro, Yokohama-shi Kanagawa 224-8558 (JP); SHIRAO Sachiko, Yokohama-shi Kanagawa 224-8558 (JP)
(74) Representative: Henkel, Breuer & Partner
(86) International application number: PCT/JP2010/056852
(87) International publication number: WO 2010/119954

(56) References cited:
- JP-A- 2001 213 722
- JP-A- 2006 257 021
- JP-A- 2007 055 990
- JP-A- 2008 189 642
- JP-A- 2008 189 642
- US-A- 5 312 933

## Description

### RELATED APPLICATIONS

The present patent application claims priority of Japanese Patent Application No. 2009-099938 filed on April 16, 2009.

### FIELD OF THE INVENTION

The present invention relates to a solid powder cosmetic and a method for producing the same, and more particularly, to an improvement of the long-lasting makeup effect in terms of makeup running and oily shining of solid powder cosmetic.

### BACKGROUND OF THE INVENTION

Solid powder cosmetic, as represented by powdery foundation or the like, have been traditionally produced using an agitation mixer such as a Henschel mixer (registered trademark), a Nauta mixer (registered trademark), a ribbon blender, or a kneader, by mixing a powder component, an oily component as a binder, and the like, cracking the mixture with a pulverizing machine such as a pulverizer, and then filling the pulverized mixture in a middle-sized dish made of a metal or a resin, or further dry press molding the pulverized mixture. These conventional methods adopt the modes of dry mixing by which mixing of a powder component and a small amount of an oily component as a binder is carried out without adding a solvent, and of dry molding by which pressurized molding in a dry powder state. Thus, the conventional methods are called as dry production methods, and have been traditionally employed.

In recent years, various methods have been developed on mixing or molding for solid powder cosmetic, so that the characteristics such as feelings of use of the solid powder cosmetic can be improved. For example, there has been suggested a method for producing a solid powder cosmetic by carrying out wet mixing, by which a powder component and an oily component are added to a volatile solvent to form a slurry, subsequently filling the mixture in a vessel in the slurry state, and removing the solvent through vacuum suction or the like to solidify the powder (wet molding). This method is referred to as a wet production method. Furthermore, investigations have also been extensively conducted on various apparatuses that are used at the time of wet mixing, by which a powder component and an oil component are mixed in a volatile solvent. For example, there have been suggested a production method of obtaining a pulverized solution by pulverizing a polymer powder in a volatile solvent using a medium agitating mill, subsequently mixing the pulverized solution and a powder such as a pigment in a wet mixing machine such as a disper to obtain a slurry, removing the volatile solvent from the slurry thus obtained to produce a dry powder, further cracking the dry powder with a pulverizing machine, and then performing dry molding to obtain a solid powder cosmetic in the solid state (Japanese patent unexamined publication (JP-A) No. H9-30926); and a method for producing a solid powder cosmetic by filling, in a vessel, the slurry obtained at the time of wet mixing using a medium agitating mill, subsequently carrying out wet molding by suction pressing, or removing the solvent from the slurry thus obtained to produce a dry powder, further cracking the dry powder with a pulverizing machine, and then performing dry molding (Japanese granted patent publication (JP-B) No. 3608778).

Along with the recent diversification and advances in consumers' needs, there have been attempts for a further enhancement in terms of usage properties such as appliability with a puff, or a satisfactory feeling of use when the cosmetic is applied on the skin, as well as in terms of productivity or the work environment. As a result, a method for producing a solid powder cosmetic by highly dispersing a wet system using a bead mill, subsequently drying the dispersion product with a flash dryer, and converting the dispersion product into fine particles (W & D production method), has been suggested (JP-A No. 2007-55990).

On the other hand, various techniques for providing a makeup cosmetic which suppresses makeup smudging and has an excellent long-lasting makeup effect, have been hitherto provided. For example, a method of absorbing sweat or sebum by incorporating a spherical porous resin powder into a makeup cosmetic (JP-A No. S55-172580) is known. However, running over time of a solid powder cosmetic such as foundation cannot be effectively prevented only by absorbing sweat or sebum.

Furthermore, techniques such as methods of adsorbing sebum components and making the long-lasting makeup effect satisfactory by incorporating activated zinc oxide and pyridoxine and/or pyridoxine derivatives into cosmetics, and by incorporating activated zinc oxide and a spherical powder of an organopolysiloxane elastomer into cosmetics (JP-A No. S62-56415 and JP-A No. H9-67223) are already known. However, although the above-described techniques can all make the long-lasting makeup effect satisfactory to a certain extent, activated zinc oxide is known to act only on fatty acids, which constitute a part of sebum components.

[Patent Literature 1] Japanese patent unexamined publication No. H9-30926
[Patent Literature 2] Japanese granted patent publication No. 3608778
[Patent Literature 3] Japanese patent unexamined publication No. 2007-55990
[Patent Literature 4] Japanese patent unexamined publication No. S55-172580
[Patent Literature 5] Japanese patent unexamined publication No. S62-56415
[Patent Literature 6] Japanese patent unexamined publication No. H9-67223

### DISCLOSURE OF THE INVENTION

### PROBLEM TO BE SOLVED BY THE INVENTION

The present invention was made in view of the related art described above, and it is an object of the present invention to provide a solid powder cosmetic having an excellent effect of preventing makeup running and oily shining, and having a satisfactory long-lasting makeup effect.

### MEANS TO SOLVE THE PROBLEM

In order to achieve the object described above, the inventors of the present invention paid attention to an amide mixture obtained by amidating a mixture of hexamethylenediamine and bisaminomethylcyclohexane with hydrogenated castor oil fatty acids, and conducted a thorough investigation. As a result, the inventors found that a solid powder cosmetic having an excellent effect of preventing makeup running and oily shining and having a satisfactory long-lasting makeup effect is obtained by incorporating the amide mixture into the formulation, thus completing the present invention.

That is, the solid powder cosmetic of the present invention is characterized by comprising: a powder component, an oily component as a binder, and an amide mixture obtained by amidating a mixture of hexamethylenediamine and bisaminomethylcyclohexane with hydrogenated castor oil fatty acids.

Furthermore, it is suitable for the solid powder cosmetic that an amount of the amide mixture is 1.0% to 15% by mass.

Furthermore, it is suitable for the solid powder cosmetic to comprise 1% to 20% by mass of spherical poly(meth)acrylate particles as the powder component.

In regard to the solid powder cosmetic, it is suitable that the spherical poly(meth)acrylate particles are particles having an average particle size of 3 to 20 µm, a specific surface area of 80 to 180 m²/g, and a most frequent pore diameter of 180 Å or larger.

Furthermore, it is suitable for the solid powder cosmetic to further comprise an organically modified clay mineral in an amount of 1.0% to 15% by mass as a total amount with the amide mixture.

It is also suitable for the solid powder cosmetic to comprise 5% to 97% by mass of fluorine compound-treated powder as the powder component.

It is also suitable for the solid powder cosmetic that the fluorine compound is 1H, 1H,2H,2H-perfluorooctyltriethoxysilane.

The method for producing a solid powder cosmetic according to the present invention is characterized by comprising: mixing step of mixing a powder component, an oily component as a binder, and an amide mixture obtained by amidating a mixture of hexamethylenediamine and bisaminomethylcyclohexane with hydrogenated castor oil fatty acids, and producing a solid powder cosmetic from the obtained powder mixture, wherein the mixing step is carried out by using a facing rotor type mixing apparatus which has a first rotor provided with plural blades and a second rotor provided with plural blades, disposed in a mixing chamber such that the first rotor and the second rotor face each other and respectively have an individual rotating shaft on the same axis line in an approximately horizontal direction, and which mixes raw materials by rotating the first rotor and the second rotor in the same or opposite direction to each other, while supplying the raw materials through a feed port on the first rotor side, and discharges the mixed raw materials through a discharge port on the second rotor side.

In regard to the method for producing a solid powder cosmetic, it is suitable that the first rotor and the second rotor of the facing rotor type mixing apparatus rotate in the opposite direction to each other..

Furthermore, the another method for producing a solid powder cosmetic according to the present invention is characterized by comprising: a slurry preparation step of mixing a powder component, an oily component as a binder, and an amide mixture obtained by amidating a mixture of hexamethylenediamine and bisaminomethylcyclohexane with hydrogenated castor oil fatty acids, in a volatile solvent to obtain a slurry; and a drying step of drying the slurry to obtain a dry powder, and producing a solid powder cosmetic from the obtained dry powder, wherein the drying step is carried out by using a drying apparatus which performs drying of the slurry by converting the slurry into fine liquid droplets by means of a mechanical shear force and blowing a dry gas to the fine liquid droplets.

In regard to the method for producing a solid powder cosmetic, it is suitable that the drying apparatus is an apparatus having a hollow-shaped housing; a shearing unit that shears a slurry by means of shearing members provided inside the housing and converts the slurry into fine liquid droplets; a supply unit that supplies the slurry to the shearing members in the housing; a gas blowing unit that blows a dry gas into the housing, and supplying the dry gas to the slurry that has been converted to fine liquid droplets by the shearing unit to bring the dry gas and the fine liquid droplets into contact; and a capture unit that captures a dry powder produced by drying the slurry.

Furthermore, in regard to the method for producing a solid powder cosmetic, it is suitable to, in the slurry preparation step, obtain a slurry by mixing the powder component and the oily component in a volatile solvent using a medium agitating mill, and cracking and/or pulverizing and/or dispersing the powder component.

### EFFECT OF THE INVENTION

According to the present invention, when an amide mixture obtained by amidating a mixture of hexamethylenediamine and bisaminomethylcyclohexane with hydrogenated castor oil fatty acids is incorporated, a solid powder cosmetic having an excellent effect of preventing makeup running and oily shining of the skin, and the like can be obtained.

### BRIEF DESCRIPTION OF THE DRAWINGS

Fig. 1 is a schematic diagram of an example of the facing rotor type mixing apparatus used in the production method (dry production method) of the present invention.
Fig. 2 is a schematic configuration diagram of an example of the apparatus configuration used in the production method (wet method) of the present invention.
Fig. 3 is a diagram showing an example of the medium agitating mill.
Fig. 4 is a diagram showing an example of the medium agitating mill.

### BEST MODE FOR CARRYING OUT THE INVENTION

Hereinafter, suitable embodiments according to the present invention will be described.

### Amide mixture

The amide mixture used in the solid powder cosmetic of the present invention is a composition obtainable by performing an amidation reaction by a conventional method, using a mixture of hexamethylenediamine and bisaminomethylcyclohexane, and hydrogenated castor oil fatty acids. When the amide mixture is incorporated into the formulation, a solid powder cosmetic having an excellent effect of preventing makeup running and oily shining and having a satisfactory long-lasting makeup effect is obtained.

There are no particular limitations on the mixing ratio of hexamethylenediamine and bisaminomethylcyclohexane, but the mixing ratio is usually, as a molar ratio, in the range of 1:9 to 9:1. Furthermore, the amides constituting the amide mixture are compounds registered at the INCI (International Nomenclature of Cosmetic Ingredients), under the names of "HEXAMETHYLENE BIS-HYDROXYSTEARAMIDE (an amide obtained by amidating hexamethylenediamine with hydrogenated castor oil fatty acids)" and "DIMETHYLENECYCLOHEXANE BIS-HYDROXYSTEARAMIDE (an amide obtained by amidating bisaminomethylcyclohexane with hydrogenated castor oil fatty acids)" (here, the hydrogenated castor oil fatty acids include hydroxystearic acid at a proportion of about 90%).

The amount of incorporation of the amide mixture in the solid powder cosmetic of the present invention is suitably 1.0% to 15% by mass relative to the total mass of the cosmetic. If the amount of incorporation of the amide mixture is less than 1.0% by mass, the effect of improving the problems of makeup running and oily shining may not be sufficiently obtained. On the other hand, if the amount of incorporation is greater than 15% by mass, the resulting solid powder cosmetic tends to have deteriorated usage properties. More preferably, the amount of incorporation of the amide mixture is 2% to 10% by mass relative to the total amount of the cosmetic.

### Powder component

There are no particular limitations on the powder component used in the present invention as long as the powder component can be generally used in cosmetic and the like. Examples of the powder component include talc, kaolin, sericite, muscovite, phlogopite, synthetic mica, lepidolite, biotite, calcined talc, calcined sericite, calcined muscovite, calcined phlogopite, vermiculite, magnesium carbonate, calcium carbonate, aluminum silicate, barium silicate, calcium silicate, magnesium silicate, strontium silicate, tungstic acid metal salts, magnesium, silica, zeolite, barium sulfate, calcined calcium sulfate (calcined gypsum), calcium phosphate, fluorapatite, ceramic powder, metal soaps (for example, zinc myristate, calcium palmitate, and aluminum stearate), boron nitride, photochromic titanium oxide (titanium dioxide sintered with iron oxide), reduced zinc oxide; organic powders (for example, a silicone elastomer powder, a silicone powder, a silicone resin-coated silicone elastomer powder, a polyamide resin powder (nylon powder), a polyethylene powder, a polystyrene powder, a styrene-acrylic acid copolymer resin powder, a benzoguanamine resin powder, a polytetrafluoroethylene powder, and a cellulose powder); inorganic white pigments (for example, titanium dioxide, and zinc oxide); inorganic red pigments (for example, iron oxide (Bengala), and iron titanate); inorganic brown pigments (for example, γ-iron oxide); inorganic yellow pigments (for example, yellow iron oxide, and ocher); inorganic black pigments (for example, black iron oxide, and lower-order titanium oxide); inorganic violet pigments (for example, mango violet and cobalt violet); inorganic green pigments (for example, chromium oxide, chromium hydroxide, and cobalt titanate); inorganic blue pigments (for example, ultramarine blue and Prussian blue); pearl pigments (for example, bismuth oxychloride, argentine, titanated mica, iron oxide-coated titanated mica, lower-order titanium oxide-coated titanated mica, photochromic titanated mica; pigments which use talc, glass, synthetic fluorphlogopite, silica, bismuth oxychloride and the like instead of mica as substrate; pigments coated with lower-order titanium oxide, colored titanium oxide, iron oxide, alumina, silica, zirconia, zinc oxide, cobalt oxide, aluminum or the like, instead of titanium oxide as the coating; pearl pigments coated on the surfaces with resin particles as functional pearl pigments (Japanese Unexamined Patent Application No. H11-92688); pearl pigments coated on the surfaces with aluminum hydroxide particles (Japanese Unexamined Patent Application No. 2002-146238); pearl pigments coated on the surfaces with zinc oxide particles (Japanese Unexamined Patent Application No. 2003-261421); pearl pigments coated on the surfaces with barium sulfate particles (Japanese Unexamined Patent Application No. 2003-61229); metal powder pigments (for example, aluminum powder, and copper powder); organic pigments such as zirconium, barium or aluminum lakes (for example, organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404; Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1); and natural coloring materials (for example, chlorophyll and β-carotene).

### Spherical poly(meth)acrylate particles

It is preferable that the solid powder cosmetic of the present invention contain spherical poly(meth)acrylate particles as the powder component in an amount of 1% to 20% by mass relative to the total amount of the cosmetic. When spherical poly(meth)acrylate particles are incorporated, the occurrence of oily shining over time can be further reduced. Furthermore, the amount of incorporation of the spherical poly(meth)acrylate particles is more preferably 1% to 10% by mass.

As the spherical poly(meth)acrylate particles, it is preferable to use porous spherical poly(meth)acrylate particles which have pores in the interior and at the surfaces, and have an average particle size of 3 to 20 µm, a specific surface area of 80 to 180 m²/g, and a most frequent pore diameter of 180 Å or larger.

The spherical poly(meth)acrylate particles used in the present invention are composed of, for example, spherical polymer particles having plural pores in the interior and at the surfaces, and are obtained by radical polymerizing a monomer mixture containing one or more selected from (meth)acrylate ester-based monomers, in the presence of a polymerization initiator and a porosifying agent. The particles can be produced by general methods for spherical polymer synthesis, such as suspension polymerization, emulsion polymerization, and soap-free polymerization. However, according to the present invention, it is particularly preferable to produce the particles by the suspension polymerization method that will be described below.

The spherical poly(meth)acrylate particles used in the present invention can be produced according to a known suspension polymerization method, using a monomer phase mixture containing at least one monomer selected from acrylate ester-based monomers and methacrylate ester-based monomers (hereinafter, collectively referred to as "(meth)acrylate ester-based monomers"), and water.

The monomer phase mixture contains a (meth)acrylate-based monomer which is polymerized while water is dispersed among the monomer molecules and turns into polymer particles having plural pores in the interior and at the surfaces, a polymerization initiator which accelerates polymerization of the (meth)acrylate-based monomer, and a comb-like polymer which disperses the water among the (meth)acrylate ester-based monomer molecules.

Examples of the (meth)acrylate ester-based monomer include α-methylene aliphatic monocarboxylic acid esters such as methyl acrylate, ethyl acrylate, n-butyl acrylate, isobutyl acrylate, propyl acrylate, n-octyl acrylate, dodecyl acrylate, 2-ethylhexyl acrylate, stearyl acrylate, 2-chloroethyl acrylate, phenyl acrylate, methyl α-chloroacrylate, methyl methacrylate, ethyl methacrylate, n-butyl methacrylate, isobutyl methacrylate, propyl methacrylate, n-octyl methacrylate, dodecyl methacrylate, 2-ethylhexyl methacrylate, stearyl methacrylate, 2-chloroethyl methacrylate, phenyl methacrylate, dimethylaminoethyl acrylate, dimethylaminoethyl methacrylate, diethylaminoethyl acrylate, and diethylaminoethyl methacrylate; (meth)acrylate derivatives such as acrylonitrile, methacrylonitrile, acrylamide, methacrylamide, 2-hydroxyethyl acrylate, 2-hydroxypropyl acrylate, 2-hydroxyethyl methacrylate, and 2-hydroxypropyl methacrylate; acrylic acid, methacrylic acid, and fumaric acid. These (meth)acrylate ester-based monomers can be used singly or as mixtures of two or more kinds. According to the present invention, it is particularly preferable to use methyl methacrylate as a monomer to obtain spherical polymethyl methacrylate (PMMA).

Furthermore, as the polymerization initiator that accelerates polymerization of the (meth)acrylate ester-based monomer, a polymerization initiator that is generally used for suspension polymerization of a (meth)acrylate ester-based monomer can be used. Examples of the polymerization initiator include peroxide-based polymerization initiators such as benzoyl peroxide, lauroyl peroxide, octanoyl peroxide, benzoyl ortho-chloroperoxide, benzoyl ortho-methoxyperoxide, methyl ethyl ketone peroxide, diisopropyl peroxydicarbonate, cumene hydroperoxide, cyclohexanone peroxide, t-butyl hydroperoxide, and diisopropylbenzene hydroperoxide; 2,2'-azobisisobutyronitrile, 2,2'-azobis(2,4-dimethylvaleronitrile), 2,2'-azobis(2-methylbutyronitrile), 2,2'-azobis(2,3,3-trimethylbutyronitrile)-2,2'-azobis(2-isopropylbutyronitrile), 1,1'-azobis(cyclohexane-1-carbonitrile), 2,2'-azobis(4-methoxy-2,4-dimethylvaleronitrile), 2-(carbamoylazo)isobutyronitrile, 4,4'-azobis(4-cyanovaleric acid), and dimethyl 2,2'-azobisisobutyrate. These polymerization initiators can be used singly or as mixtures of two or more kinds.

Benzoyl peroxide, lauroyl peroxide, 2,2'-azobisisobutyronitrile, and 2,2'-azobis(2,4-dimethylvaleronitrile) are suitable from the viewpoint that these compounds are easily dissolved in the monomer, and handling thereof is easy.

The amount of incorporation of the polymerization initiator varies depending on the monomer used, but is usually 0.01 to 1.00 parts by mass relative to 100 parts by mass of the monomer.

The comb-like polymer has many trifurcation points with linear side chains in the main chain, and usually has a weight average molecule of 2,000 to 100,000.

There are no particular limitations on the comb-like polymer as long as the polymer has a hydrophilic portion and a hydrophobic portion in the molecule. However, from the viewpoint that the water dispersed among the monomer molecules can be easily stabilized in a particulate form, a comb-like polymer in which plural side chains constituting the hydrophobic portion are graft-bonded to the main chain having the hydrophilic portion is suitable. Examples of the comb-like polymer that can be used include a poly(ethyleneimine) which has one or more poly(carbonyl-C3-C6 alkyleneoxy) chains, while each chain has three to eighty carbonyl-C3-C6 alkyleneoxy groups and is bonded to poly(ethyleneimine) through an amide or salt-crosslinking group; an acid salt of such a polyethyleneimine; and a reaction product between a poly(lower alkylene)imine and a polyester having a free carboxylic acid group, in which at least two or more polyester chains are bonded to each poly(lower alkylene)imine chain. As such a comb-like polymer, for example, the "SOLSPERSE" series that are commercially available from Lubrizol Corp. in the United Kingdom, can be used.

The comb-like polymer material preferably has an acid value of 20 to 80. If the acid value is less than 20, the polymer particles may not all have plural pores formed in the interiors and at the surfaces of the polymer particles, and if the acid value is greater than 80, polymerization becomes unstable, and the polymer may not be obtained as a particulate material. The acid value can be measured as the number of milligrams of KOH required to neutralize free carboxylic acids contained in 1 g of the comb-like polymer, according to JIS K0070.

The amount of incorporation of the comb-like polymer material is suitably 0.01 to 3.00 parts by mass relative to 100 parts by mass of the monomer. If the amount of incorporation is less than 0.01 parts by mass, the polymer particles may not have plural pores formed in the interior and at the surfaces. On the other hand, if the comb-like polymer is incorporated in an amount of even greater than 3 parts by mass, a pore forming effect (ease of forming pores) corresponding to the amount of incorporation may not be obtained, while there is a risk that the comb-like polymer material may decrease the purity of the polymer and impair the properties of the polymer.

Furthermore, in addition to the components described above, a crosslinking agent for the monomer, another monomer or oligomer, and the like can be added to the monomer phase mixture, to the extent that the effect of the invention is not impaired.

Such a crosslinking agent may be a compound having two or more polymerizable double bonds, and examples include aromatic divinyl compounds such as divinylbenzene, divinylnaphthalene, and derivatives thereof; diethylenic carboxylic acid esters such as ethylene glycol dimethacrylate, triethylene glycol triacrylate, and trimethylolpropane triacrylate; and compounds having three or more divinyl compounds and vinyl groups, such as N,N-divinylaniline, divinyl ether, and divinyl sulfite. These compounds can be used singly or as mixtures of two or more kinds.

Furthermore, examples of the other monomer or oligomer that can be used include styrene and derivatives thereof, and vinyl esters such as vinyl butyrate.

The water for dispersing the monomer phase mixture can contain a dispersion stabilizer or a surfactant for aqueous media.

As the dispersion stabilizer, a dispersion stabilizer that is generally used in suspension polymerization of polymers can be used, and examples that can be used include water-soluble polymers such as methyl cellulose, hydroxyethyl cellulose, and polyvinyl alcohol; and sparingly water-soluble inorganic salts such as tribasic calcium phosphate, magnesium hydroxide, magnesium pyrophosphate, barium sulfate, calcium carbonate, and silica. Particularly, from the viewpoints that the dispersion stabilizer can be easily removed from the polymer after polymerization, and that polymer particles can be polymerized with a narrow particle size distribution, a sparingly water-soluble inorganic salt having a solubility in water at normal temperature of about 3 mg or less is preferable, and tribasic calcium phosphate having a solubility of 2.5 mg is suitable.

The dispersion stabilizer is usually incorporated into water at a proportion of 0.1 to 20.0 parts by mass relative to 100 parts by mass of the polymer particles that are obtained.

As the surfactant for aqueous media, a surfactant for aqueous media that is generally used in suspension polymerization of polymers can be used. From the viewpoint that polymer particles can be polymerized with a narrow particle size distribution, anionic surfactants such as sodium dodecyl sulfate, sodium dodecyl benzenesulfonate, sodium polyoxyethylene lauryl ether sulfate, and sodium diethylsulfosuccinate are particularly preferred. Usually, such a surfactant for aqueous media is incorporated at a concentration of 0.005% to 0.3% by mass based on water.

Next, a specific method for producing spherical poly(meth)acrylate particles will be described.

The monomer phase mixture and water are respectively adjusted to predetermined amounts of incorporation in separate vessels and are mixed. That is, on one side, a (meth)acrylate-based monomer, a comb-like polymer, a polymerization initiator, a crosslinking agent, another monomer, an oligomer and the like are mixed and agitated at predetermined proportions as a monomer phase mixture. As a mixing and agitating unit that may be used at this time, a general mixer or a homogenizer can be used, but it is preferable to employ a mixing and agitating unit that would make the system thoroughly uniform. Furthermore, when there is a risk that the temperature of the monomer phase mixture may increase as a result of mixing and agitation, and polymerization of the (meth)acrylate ester-based monomer may be initiated, it is preferable to mix and agitate the system while suppressing the temperature increase using a cooling unit or the like.

On the other side, as a material for aqueous phase, a dispersion stabilizer and a surfactant for aqueous media are added to water at predetermined proportions, and the mixture is mixed and agitated. In this case as well, a general mixer or a homogenizer can be used as a mixing and agitating unit to be used, and it is preferable to employ a mixing and agitating unit that would make the system thoroughly uniform.

Thereafter, the monomer phase mixture is poured into the water that has been prepared as described above, and the resulting mixture is mixed and agitated with a homogenizer or the like, to obtain a suspension liquid (aqueous phase/monomer phase/aqueous phase emulsion). At this time, the particle size of the monomer phase, that is, the particle size of the polymer particles having plural pores in the interior and at the surfaces, can be easily adjusted by varying the conditions for agitation such as the duration of agitation and the speed of rotation while using a homogenizer as an agitating unit.

During the production of the spherical poly(meth)acrylate particles used in the present invention, it is preferable to adjust the particle size such that the average particle size reaches 3 to 20 µm. If the average particle size is less than 3 µm, when the spherical poly(meth)acrylate particles are incorporated into a cosmetic, sufficient sebum absorbing properties are not exhibited, and an excellent long-lasting makeup effect cannot be obtained. If the average particle size is larger than 20 µm, the feeling of use of the cosmetic to which the spherical poly(meth)acrylate particles are incorporated, tends to be deteriorated.

The suspension liquid is introduced into a heating apparatus such as an autoclave, and the suspension liquid is heated, while agitated, to thereby carry out polymerization of the monomer phase. The polymerization product thus obtained is filtered, and the filter cake is washed with water and then dried. Thus, spherical poly(meth)acrylate particles having plural pores in the interior and at the surfaces can be obtained.

Furthermore, if necessary, a process of removing the dispersion stabilizer before the washing can also be carried out.

In the spherical poly(meth)acrylate particles that may be obtained as described above, pores generated using, as a template, a comb-like polymer that is finely dispersed in the monomer phase by mixing, are formed numerously in the interiors and at the surfaces of the particles.

In the production of the spherical poly(meth)acrylate particles used in the present invention, a known material which is known as a porogen can be used, in addition to the comb-like polymer described above, as the material that forms plural pores (porosification) in the interiors and at the surfaces of the polymer particles. Examples of the porogen include toluene, isooctane, methyl isobutyl ketone, calcium carbonate, tricalcium phosphate, and various linear polymers. These compounds can be used singly or in combination of two or more kinds. Definitely, the method for producing spherical poly(meth)acrylate particles can be appropriately modified in accordance with the porosifying material used, regardless of the method described above.

Furthermore, the pore diameter or shape of the pores in the interiors and at the surfaces of the spherical poly(meth)acrylate particles, and the specific surface area of the particles can be regulated by known techniques in accordance with the type of the porosifying material. That is, the pore diameter of the pores and the specific surface area of the spherical poly(meth)acrylate particles can be regulated by appropriately selecting the method for application of the porosifying material to the polymer particle synthesis process (including the conditions for agitation), the amount of application or the like, in accordance with the characteristics of the material used.

According to the present invention, particularly, spherical poly(meth)acrylate particles formed so as to have a specific surface area of the particles of 80 to 180 m²/g are suitably used.

If the specific surface area is smaller than 80 m²/g, the effect of sebum absorption by the pores is not sufficiently obtained, and an excellent long-lasting makeup effect may not be obtained. Furthermore, if the specific surface area is larger than 180 m²/m, the pores are so densely present that the sebum absorption capacity of the polymer particles themselves may be decreased, and also, the usage properties may be deteriorated.

For the spherical poly(meth)acrylate particles used in the present invention, the most frequent pore diameter of the pores in the interiors and at the surfaces of the particles is preferably 180 Å or larger. When the most frequent pore diameter is 180 Å or larger, the oil absorption (oleic acid, which is a sebum component) of the particles reaches 100 to 300 ml/100 g, and makeup smudging due to sebum can be prevented to a large extent. If the most frequent pore diameter is smaller than 180 Å, since the pores are small, the oil absorption efficiency and the oil absorption are decreased, and a sufficient long-lasting makeup effect may not be obtained.

The most frequent pore diameter described above has no particular upper limit. However, if the strength of the particles, the feeling of use when the particles are incorporated into a cosmetic, and the like are to be considered, the most frequent pore diameter is preferably adjusted to about 180 to 400 Å.

According to the present invention, commercially available powders may also be used as the spherical poly(meth)acrylate particles having pores in the interior and at the surfaces, as long as the powders have the particle size and the specific surface area described above. An example of such a powder that can be suitably used may be "TECHPOLYMER MBP-8HP" manufactured by Sekisui Chemical Co., Ltd.

### Fluorine compound-treated powder

In regard to the powder component used in the present invention, a powder component that is not surface-treated may be used, or a powder component that has been surface-treated with a silicone, a fluorine compound, a fatty acid soap or the like may also be used. However, it is particularly preferable to incorporate a powder component that has been surface-treated with a fluorine compound.

When a fluorine compound-treated powder is incorporated into the solid powder cosmetic, water repellency and oil repellency are imparted to the solid powder cosmetic, and the solid powder cosmetic does not easily dissolve in sweat or sebum.

Examples of the fluorine compound that is used to treat the powder surface include perfluoroalkyl phosphoric acid ester-diethanolamine salts, perfluoroalkylsilanes, perfluoroalkyl ethyl acrylates; and compounds having perfluoropolyether groups, such as perfluoropolyether dialkyl phosphoric acid and salts thereof, perfluoropolyether dialkyl sulfate and salts thereof, perfluoropolyether dialkyl carboxylic acid and salts thereof. Particularly, fluorine compounds having any one perfluoroalkyl group selected from CF₂-, CF₃-, CF₃CF₂-, and CF₂CF₂- in the molecule are suitable.

As the fluorine compound, 1H,1H,2H,2H-perfluorooctyltriethoxysilane represented by the following formula (I) can be particularly suitably used.

CF₃CF₂CF₂CF₂CF₂CF₂CH₂CH₂Si(OEt)₃ (I)

Furthermore, specific examples of other fluorine compounds include fluorine compounds represented by the following formulas (II) to (IV). 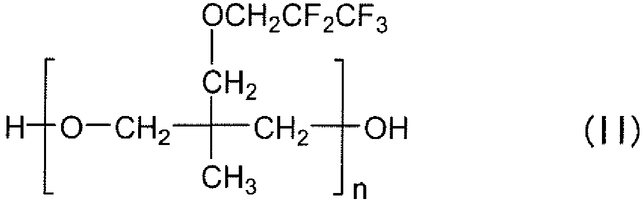 wherein in the formula (II), n represents an integer from 3 to 25. 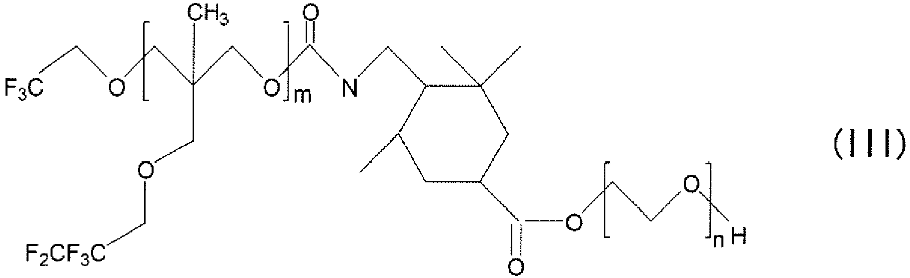 wherein in the formula (III), m and n each represent an integer from 5 to 20.

X-CF₂O-(CF₂CF₂O)ₚ-(CF₂O)_{q}-CF₂-X (IV)

wherein in the formula (IV), X represents any one of CH₂OH, CO-NH-C₁₈H₃₇, and CH₂C-(OCH₂CH₂)p-OPO(OH)₂; and the p/q ratio represents an integer from 0.5 to 3.0.

Furthermore, a surface treatment of the powder component may be carried out by using the fluorine compound and another hydrophobization treating agent in combination. Specific examples of other treating agents include an acrylic silicone compound represented by the following formula (V). 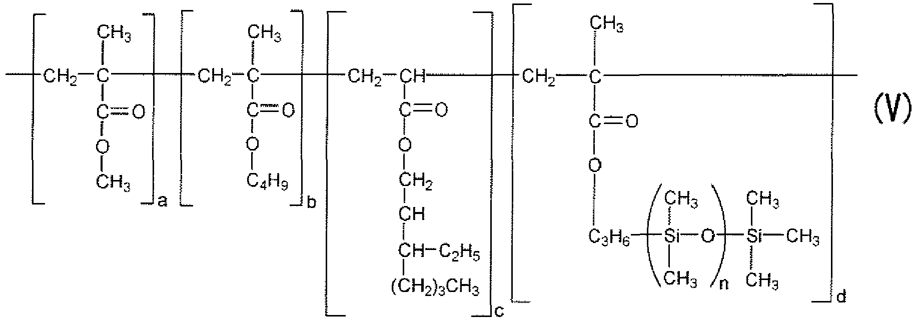 wherein n represents an integer; a, b, c and d represent the respective molar ratios in the copolymer, and are not necessarily zero; and d is from 40% by mole to 60% by mole.

The surface treatment of the powder component with a fluorine compound can be carried out according to a conventional method.

For example, a surface-treated powder can be produced by bringing the fluorine compound of the formula (I) (and the acrylic silicone compound of the formula (V)), which is in the form of a solution having the fluorine compound dissolved in an appropriate solvent or in the form of the liquid of the fluorine compound itself, into contact with the powder, and then heating the resultant at 100°C to 150°C, and preferably 120°C to 140°C, for 1 to 12 hours, and preferably 3 to 9 hours.

For the heating atmosphere, the heating may be carried out in air, which is a moisture-containing atmosphere, or in another gas containing moisture at least to the extent that is contained in air. In addition to these, the heating may also be carried out by a method of producing the surface-treated powder in an atmosphere that does not contain moisture, and then heating the surface-treated powder while adding moisture during the treatment (during heating); or by a method of adding a solution containing one or more metal salts of aluminum (III), tin (II), tin (IV), iron (III) or titanium (III) in a small amount of water, simultaneously with or previously to the surface treating agent (the fluorine compound of formula (I) (and the acrylic silicone compound of formula (V)). Specific examples of the metal salts include aluminum chloride, stannic chloride, and ferric chloride (including hydrates thereof).

When the fluorine compound of the formula (I) (and the acrylic silicone compound of the formula (V)) is brought into contact with the powder in the form of a solution in which the fluorine compound is dissolved in an appropriate solvent, for example, a solution containing 0.3% to 50% by mass of the fluorine compound in a solvent such as an alcohol, water, hexane, cyclohexane or toluene is prepared. The powder is dispersed in the solution, and then the dispersion is heated. Thereby, the solvent is evaporated, and at the same time, the fluorine compound of the formula (I) (and the acrylic silicone compound of the formula (V)) is polymerized on the surfaces of the powder. Thus, a treated powder is obtained. This process can be carried out using a Henschel mixer, a Lödige mixer, a kneader, a medium agitating mill (bead mill or the like), or the like. As the apparatus used in the heating, an electric furnace, a tunnel furnace, a roller hearth kiln, a rotary kiln, or the like can be used.

When the fluorine compound of the formula (I) (and the acrylic silicone compound of the formula (V)) is directly brought into contact with the powder without dissolving the compound in a solvent, the fluorine compound is brought into contact with the powder using an appropriate mixing machine such as, for example, a rotary ball mill, a vibratory ball mill, a planetary ball mill, a sand mill, an attriter, a pug mill, a pony mixer, a planetary mixer, a Raikai mixer, a Henschel mixer, or the like. Thus, a treated powder is obtained.

In the solid powder cosmetic of the present invention, the amount of incorporation of the fluorine compound-treated powder is preferably 5% to 97% by mass, and more preferably 20% to 75% by mass, relative to the total amount of the powder cosmetic. If the amount of incorporation of the fluorine compound-treated powder is less than 5% by mass, the effect of incorporating the fluorine compound-treated powder may not be sufficiently obtained. If the amount of incorporation is greater than 97% by mass, the feeling of use may be deteriorated.

The solid powder cosmetic of the present invention may be produced by mixing a fluorine compound-treated powder that has been treated in advance with an oily component together with other powder components. Alternatively, an untreated powder component may be mixed with an oily component together with the fluorine compound, so that the surfaces of the powder component may be treated with the fluorine compound at the time of mixing. These can be appropriately selected according to the types of the various components or the production process; however, usually, in the case of a method of mixing a powder component and an oily component in a dry system (dry production method), the former method is adequate, and in the case of a method of dispersing a powder component and an oily component to obtain a slurry in a wet system, and then drying the slurry (wet production method), the latter method is adequate. In the production process, when the powder component is surface-treated by adding a fluorine compound to a mixture, the amount of addition of the fluorine compound is preferably 0.1% to 10% by mass, and more preferably 0.5% to 5% by mass, relative to the total amount of the cosmetic.

### Oily component

There are no particular limitations on the oily component used in the present invention as long as it is an oily component that can be generally used. Specific examples include liquid oils and fats, solid oils and fats, waxes, hydrocarbons, higher fatty acids, and higher alcohols.

Examples of the liquid oils and fats include avocado oil, camellia oil, turtle oil, macadamia nut oil, corn oil, mink oil, olive oil, rapeseed oil, egg yolk oil, sesame oil, persic oil, wheat germ oil, sasanqua oil, castor oil, linseed oil, safflower oil, cotton seed oil, perilla oil, soybean oil, peanut oil, tea seed oil, kaya oil, rice bran oil, Chinese tung oil, Japanese tung oil, jojoba oil, germ oil, and triglycerin.

Examples of the solid oils and liquids include cacao fat, coconut oil, horse fat, hardened coconut oil, palm oil, beef tallow, mutton tallow, hardened beef tallow, palm kernel oil, lard, beef bone fat, Japan wax kernel oil, hardened oil, neat's foot oil, Japan wax, and hardened castor oil.

Examples of the waxes include beeswax, candelilla wax, cotton wax, carnauba wax, bayberry wax, insect wax, whale wax, montan wax, rice bran wax, lanolin, kapok wax, lanolin acetate, liquid lanolin, sugar cane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, and POE hydrogenated lanolin alcohol ether.

Examples of the hydrocarbon oils include liquid paraffin, ozokerite, squalane, pristane, paraffin, ceresin, squalene, petrolatum, and microcrystalline wax.

Examples of the higher fatty acids include lauric acid, myristic acid, palmitic acid, stearic acid, behenic acid, oleic acid, undecylenic acid, toluic acid, isostearic acid, linolic acid, linoleic acid, eicosapentaenoic acid (EPA), and docosahexaenoic acid (DHA).

Examples of the higher alcohols include straight chain alcohols (for example, lauryl alcohol, cetyl alcohol, stearyl alcohol, behenyl alcohol, myristyl alcohol, oleyl alcohol, and cetostearyl alcohol); and branched chain alcohols (for example, monostearyl glycerin ether (batyl alcohol), 2-decyltetradecinol, lanolin alcohol, cholesterol, phytosterol, hexyldodecanol, isostearyl alcohol, and octyldodecanol).

Examples of the synthetic ester oils include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glyceryl di-2-heptylundecanoate, trimethylolpropane tris(2-ethylhexanoate), trimethylolpropane triisostearate, pentaerythritol tetra(2-ethylhexanoate), glyceryl tri(2-ethylhexanoate), glyceryl trioctanoate, glyceryl triisopalmitate, trimethylolpropane triisostearate, cetyl 2-ethylhexanoate, 2-ethylhexyl palmitate, glyceryl trimyristate, tri-2-heptylundecanoic acid glyceride, castor oil fatty acid methyl esters, oleyl oleate, acetoglyceride, 2-heptyl undecyl palmitate, diisobutyl adipate, N-lauroyl-L-glutamic acid 2-octyl dodecyl ester, di-2-heptylundecyl adipate, ethyl laurate, di-2-ethylhexyl sebacate, 2-hexyl decyl myristate, 2-hexyl decyl palmitate, 2-hexyl decyl adipate, diisopropyl sebacate, 2-ethylhexyl succinate, and triethyl citrate.

### Organically modified clay mineral

It is preferable that the solid powder cosmetic of the present invention further contain an organically modified clay mineral. When an organically modified clay mineral is further incorporated, in addition to the amide mixture, to the solid powder cosmetic, the occurrence of makeup running over time in particular can be reduced. Furthermore, the amount of incorporation of the organically modified clay mineral is preferably, as a sum with the amount of incorporation of the amide mixture, 1.0% to 15% by mass relative to the total amount of the cosmetic.

The organically modified clay mineral used in the present invention may be a clay mineral (for example, montmorillonite, saponite, hectorite, and bentonite) in which an exchangeable cation interposed between the crystal layers has been substituted with an organic polar compound or an organic cation. Specific examples include dimethyldistearylammonium hectorite (= quaternium-18 hectorite), dimethyldistearylammonim bentonite (= quaternium-18 bentonite), dioctadecyldimethylammonium salt-modified montmorillonite, octadecyldimethylbenzylammonium salt-modified montmorillonite, and dihexadecyldimethylammonium salt-modified montmorillonite. Organically modified clay minerals that may be used in the present invention are sold on the market as, for example, "BENTONE 38" (quaternium-18 hectorite), "BENTONE 34" (= quaternium-18 bentonite) (all manufactured by Rheox, Inc.), "CLAYTONE SO" (all manufactured by Southern Clay Products, Inc.), TIXOGEL series (manufactured by Süd-Chemie Group) and the like, and are commercially available. The organically modified clay minerals can be used singly or in combination of two or more kinds.

Apart from using a clay mineral that has been organically modified in advance, an unmodified clay mineral such as synthetic smectite (aluminum magnesium silicate), and a cationic surfactant may be separately incorporated into a composition, so as to organically modify the clay mineral during the process for producing a solid powder cosmetic.

### Other components

Furthermore, the solid powder cosmetic according to the present invention can be produced by a routine method in accordance with the desired formulation, by appropriately incorporating other components, for example, an ester, an anionic surfactant, a cationic surfactant, an amphoteric surfactant, a nonionic surfactant, a moisturizer, a water-soluble polymer, a thickening agent, a film-forming agent, an ultraviolet absorber, a metal ion sequestering agent, a lower alcohol, a polyhydric alcohol, a sugar, an amino acid, an organic amine, a polymer emulsion, a pH adjusting agent, a skin nutrient, a vitamin, an antioxidant, an antioxidant aid, a fragrance, and water as necessary, to the extent that the effects of the present invention are not impaired. Specific components that can be incorporated will be listed below, and a solid powder cosmetic can be produced by incorporating the above-described essential components to be incorporated, and any one kind or two or more kinds of the components described below.

Examples of the anionic surfactant include fatty acid soaps (for example, sodium laurate, and sodium palmitate); higher alkyl sulfuric acid ester salts (for example, sodium lauryl sulfate, and potassium lauryl sulfate); alkyl ether sulfuric acid ester salts (for example, triethanolamine POE-lauryl sulfate, and sodium POE-lauryl sulfate); N-acyl sarcosine acids (for example, sodium lauroyl sarcosine); higher fatty acid amide sulfonates (for example, sodium N-myristoyl-N-methyltaurin, sodium coconut oil fatty acid methyltauride, and sodium laurylmethyltauride); phosphoric acid ester salts (for example, sodium POE-oleyl ether phosphate, and POE-stearyl ether phosphoric acid); sulfosuccinates (for example, sodium di-2-ethylhexylsulfosuccinate, sodium monolauroylmonoethanolamide polyoxyethylene sulfosuccinate, and sodium lauryl polypropylene glycol sulfosuccinate); alkylbenzene sulfonates (for example, sodium linear dodecyl benzenesulfonate, triethanolamine linear dodecyl benzenesulfonate, and linear dodecyl benzenesulfonic acid); higher fatty acid ester sulfuric acid ester salts (for example, sodium hardened coconut oil fatty acid glycerin sulfate); N-acyl glutamates (for example, monosodium N-lauroyl glutamate, disodium N-stearoyl glutamate, and monosodium N-myristoyl-L-glutamate); sulfated oils (for example, Turkey red oil); POE-alkyl ether carboxylic acids; POE-alkylaryl ether carboxylates; α-olefin sulfonates; higher fatty acid ester sulfonates; secondary alcohol sulfuric acid ester salts; higher fatty acid alkylolamide sulfuric acid ester salts; sodium lauroyl monoethanolamidosuccinate; triethanolamine N-palmitoyl aspartate; and casein sodium.

Examples of the cationic surfactant include alkyltrimethylammonium salts (for example, stearyltrimethylammonium chloride, and lauryltrimethylammonium chloride); alkylpyridinium salts (for example, cetylpyridinium chloride); distearyldimethylammonium chloride dialkyldimethylammonium salts; poly(N,N'-dimethyl-3,5-methylenepiperidinium) chloride; alkyl-quaternary ammonium salts; alkyldimethylbenzylammonium salts; alkylisoquinolinium salts; dialkylmorpholinium salts; POE-alkylamines; alkylamine salts; polyamine fatty acid derivatives; amyl alcohol fatty acid derivatives; benzalkonium chloride; and benzetonium chloride.

Examples of the amphoteric surfactant include imidazoline-based amphoteric surfactants (for example, sodium 2-undecyl-N,N,N-(hydroxyethylcarboxymethyl)-2-imidazoline, and 2-cocoyl-2-imidazolinium hydroxide 1-carboxyethyloxy disodium salt); and betaine-based surfactants (for example, 2-heptadecyl-N-carboxymethyl-N-hydroxyethylimidazolinium betaine, lauryldimethylaminoacetic acid betaine, alkylbetaine, amidobetaine, and sulfobetaine).

Examples of lipophilic nonionic surfactants include sorbitan fatty acid esters (for example, sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexanoate, and diglycerol sorbitan tetra-2-ethylhexanoate); glycerin polyglycerin fatty acids (for example, mono-cotton seed oil fatty acid glycerin, glyceryl monoerucate, glyceryl sesquioleate, glyceryl monostearate, glyceryl α,α'-oleate pyroglutamate, and glyceryl monostearate malate); propylene glycol fatty acid esters (for example, propylene glycol monostearate); hardened castor oil derivatives; and glycerin alkyl ethers.

Examples of hydrophilic nonionic surfactants include POE-sorbitan fatty acid esters (for example, POE-sorbitan monooleate, POE-sorbitan monostearate, POE-sorbitan monooleate, and POE-sorbitan tetraoleate); POE-sorbite fatty acid esters (for example, POE-sorbite monolaurate, POE-sorbite monooleate, POE-sorbite pentaoleate, and POE-sorbite monostearate); POE-glycerin fatty acid esters (for example, POE-monooleates such as POE-glycerin monostearate, POE-glycerin monoisostearate, and POE-glycerin triisostearate); POE-fatty acid esters (for example, POE-distearate, POE-monodioleate, and ethylene glycol distearate); POE-alkyl ethers (for example, POE-lauryl ether, POE-oleyl ether, POE-stearyl ether, POE-behenyl ether, POE-2-octyldodecyl ether, and POE-cholestanol ether); Pluronic types (for example, Pluronic); POE·POP-alkyl ethers (for example, POE·POP-cetyl ether, POE·POP-2-decyltetradecyl ether, POE-POP-monobutyl ether, POE·POP-hydrogenated lanolin, and POE·POP-glycerin ether); tetra-POE·tetra-POP-ethylenediamine condensates (for example, Tetronic); POE-castor oil hardened castor oil derivatives (for example, POE-castor oil, POE-hardened castor oil, POE-hardened castor oil monoisostearate, POE-hardened castor oil triisostearate, POE-hardened castor oil monopyroglutamate monoisostearate diester, and POE-hardened castor oil maleate); POE-beeswax lanolin derivatives (for example, POE-sorbite beeswax); alkanolamides (for example, coconut oil fatty acid diethanolamide, lauric acid monoethanolamide, and fatty acid isopropanolamide); POE-propylene glycol fatty acid esters; POE-alkylamines; POE-fatty acid amides; sucrose fatty acid esters; alkylethoxydimethylamine oxide; and trioleylphosphoric acid.

Examples of the moisturizer include polyethylene glycol, propylene glycol, glycerin, 1,3-butylene glycol, xylytol, sorbitol, maltitol, chondroitin sulfate, hyaluronic acid, mucoitin sulfate, caronic acid, atherocollagen, cholesteryl 12-hydroxystearate, sodium lactate, bile acid salts, dl-pyrrolidonecarboxylic acid salts, alkylene oxide derivatives, short-chain soluble collagen, diglycerin (EO) PO adducts, Rosa roxburghii extract, Achillea milefolium extract, and melilot extract.

Examples of natural water-soluble polymers include plant-derived polymers (for example, gum arabic, tragacanth gum, galactan, guar gum, carob gum, karaya gum, carrageenan, pectin, agar, quince seed (marmelo), algae colloid (brown algae extract), starch (rice, corn, potato, and wheat), and glycyrrhizic acid); microorganism-derived polymers (for example, xanthan gum, dextran, succinoglucan, and pullulan); and animal-derived polymers (for example, collagen, casein, albumin, and gelatin).

Examples of semisynthetic water-soluble polymers include starch-based polymers (for example, carboxymethyl starch, and methylhydroxypropyl starch); cellulose-based polymers (for example, methyl cellulose, ethyl cellulose, methylhydroxypropyl cellulose, hydroxyethyl cellulose, sodium cellulose sulfate, hydroxypropyl cellulose, carboxymethyl cellulose, carboxymethyl cellulose sodium, crystalline cellulose, and cellulose powder); and alginic acid-based polymers (for example, sodium alginate, and alginic acid propylene glycol ester).

Examples of synthetic water-soluble polymers include vinyl-based polymers (for example, polyvinyl alcohol, polyvinyl methyl ether, polyvinylpyrrolidone, and carboxyvinyl polymers); polyoxyethylene-based polymers (for example, polyoxyethylene-polyoxypropylene copolymers, such as Polyethylene Glycol 20,000, 40,000, 60,0000); acrylic polymers (for example, sodium polyacrylate, polyethyl acrylate, and polyacrylamide); polyethyleneimine; and cationic polymers.

Examples of the thickening agent include gum arabic, carrageenana, karaya gum, tragacanth gum, carob gum, quince seed (marmelo), casein, dextrin, gelatin, sodium pectinate, sodium alginate, methyl cellulose, ethyl cellulose, CMC, hydroxyethyl cellulose, hydroxypropyl cellulose, PVA, PVM, PVP, sodium polyacrylate, carboxyvinyl polymer, locust bean gum, guar gum, tamarind gum, dialkyldimethylammonium sulfate cellulose, xanthan gum, aluminum magnesium silicate, bentonite, hectorite, AlMg silicate (veegum), laponite, and silicic anhydride.

Examples of the ultraviolet absorber include benzoic acid-based ultraviolet absorbers (for example, para-aminobenzoic acid (hereinafter, abbreviated to PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA ethyl ester); anthranilic acid-based ultraviolet absorbers (for example, homomenthyl-N-acetyl anthranilate); salicylic acid-based ultraviolet absorbers (for example, amyl salicylate, menthyl salicylate, homomenthyl salicylate, octyl salicylate, phenyl salicylate, benzyl salicylate, and p-isopropanolphenyl salicylate); cinnamic acid-based ultraviolet absorbers (for example, octyl methoxycinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, octyl-p-methoxycinnamate (2-ethylhexyl-p-methoxycinnamate), 2-ethoxyethyl-p-methoxycinnamate, cyclohexyl-p-methoxycinnamate, ethyl-α-cyano-β-phenyl cinnamate, 2-ethylhexyl-α-cyano-β-phenyl cinnamate, and glyceryl mono-2-ethylhexanoyl di-para-methoxycinnamate); benzophenone-based ultraviolet absorbers (for example, 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxybenzophenone, and 4-hydroxy-3-carboxybenzophenone); 3-(4'-methylbenzylidene)-d,1-camphor; 3-benzylidene-d,1-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenylbenzotriazole; dibenzalazine; dianisoylmethane; 4-methoxy-4'-t-butyldibenzoylmethane; 5-(3,3-dimethyl-2-norbornylidene)-3-pentan-2-one, dimorpholinopyridazino; 2-ethylhexyl-2-cyano-3,3-diphenyl acrylate; and 2,4-bis-{[4-(2-ethylhexyloxy)-2-hydroxy]-phenyl}-6-{4-methoxyphenyl)-(1,3,5)-triazine.

Examples of the metal ion sequestering agent include 1-hydroxyethane-1,1-diphosphonic acid, 1-hydroxyethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, and trisodium ethylenediaminehydroxyethyl triacetate.

Examples of the lower alcohol include ethanol, propanol, isopropanol, isobutyl alcohol, and t-butyl alcohol.

Examples of the polyhydric alcohol include dihydric alcohols (for example, ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, and octylene glycol); trihydric alcohols (for example, glycerin, and trimethylolpropane); tetrahydric alcohols (for example, pentraerythritols such as 1,2,6-hexanetriol); pentahydric alcohols (for example, xylytol); hexahydric alcohols (for example, sorbitol, and mannitol); polyhydric alcohol polymers (for example, diethylene glycol, dipropylene glycol, triethylene glycol, polypropylene glycol, tetraethylene glycol, diglycerin, polyethylene glycol, triglycerin, tetraglycerin, and polyglycerin); dihydric alcohol alkyl ethers (for example, ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, and ethylene glycol dibutyl ether); dihydric alcohol alkyl ethers (for example, diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether); dihydric alcohol ether esters (for example, ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate); glycerin monoalkyl ethers (for example, chimyl alcohol, selachyl alcohol, and batyl alcohol); sugar alcohols (for example, sorbitol, maltitol, maltotriose, mannitol, sucrose, erythritol, glucose, fructose, starch-decomposed sugar, maltose, xylytose, and starch-decomposed sugar reduced alcohol); glysolids; tetrahydrofurfuryl alcohol; POE-tetrahydrofurfuryl alcohol; POP-butyl ether; POP·POE-butyl ether; tripolyoxypropylene glycerin ether; POP-glycerin ether; POP-glycerin ether phosphate; POP·POE-pentane erythritol ether; and polyglycerin.

Examples of monosaccharides include trioses (for example, D-glycerylaldehyde, and dihydroxyacetone); tetroses (for example, D-erythrose, D-erythrulose, D-threose, and erythritol); pentoses (for example, L-arabinose, D-xylose, L-lyxose, D-arabinose, D-ribose, D-ribulose, D-xylulose, and L-xylulose); hexoses (for example, D-glucose, D-talose, D-psicose, D-galactose, D-fructose, L-galactose, L-mannose, and D-tagatose); heptoses (for example, aldoheptose, and heptulose); octoses (for example, octulose); deoxy sugars (for example, 2-deoxy-D-ribose, 6-deoxy-L-galactose, and 6-deoxy-L-mannose); amino sugars (for example, D-glucosamine, D-galactosamine, sialic acid, aminouronic acid, and muramic acid); and uronic acids (for example, D-glucuronic acid, D-mannuronic acid, L-guluronic acid, D-galacturonic acid, and L-iduronic acid).

Examples of oligosaccharides include sucrose, gentianose, umbelliferose, lactose, planteose, isolychnoses, α,α-threhalose, raffinose, lychnoses, umbilicin, and stachyose verbascoses.

Examples of polysaccharides include cellulose, quince seed, chondroitin sulfate, starch, galactan, dermatan sulfate, glycogen, gum arabic, heparan sulfate, hyaluronic acid, tragacanth gum, keratan sulfate, chondroitin, xanthan gum, mucoitin sulfate, guar gum, dextran, keratosulfuric acid, locust bean gum, succinoglucan, and caronic acid.

Examples of the amino acid include neutral amino acids (for example, threnonine, and cysteine); and basic amino acids (for example, hydroxylysine). Furthermore, examples of amino acid derivatives include sodium acylsarcosine (sodium lauroylsarcosine), acyl glutamic acid salts, sodium acyl β-alanine, glutathione, and pyrrolidonecarboxylic acid).

Examples of the organic amine include monoethanolamine, diethanolamine, triethanolamine, morpholine, triisopropanolamine, 2-amino-2-methyl-1,3-propanediol, and 2-amino-2-methyl-1-propanol.

Examples of the polymer emulsion include an acrylic resin emulsion, a polyethyl acrylate emulsion, an acrylic resin liquid, a polyacrylalkyl ester emulsion, a polyvinyl acetate resin emulsion, and natural rubber latex.

Examples of the pH adjusting agent include buffers such as lactic acid-sodium lactate, citric acid-sodium citrate, and succinic acid-sodium succinate.

Examples of vitamins include vitamins A, B1, B2, B6, C, E and derivatives thereof, pantothenic acid and derivatives thereof, and biotin.

Examples of the antioxidant include tocopherols, dibutylhydroxytoluene, butylhydroxyanisole, and gallic acid esters.

Examples of the antioxidant aid include phosphoric acid, citric acid, ascorbic acid, maleic acid, malonic acid, succinic acid, fumaric acid, cephalin, hexametaphosphate, phytic acid, and ethylenediaminetetraacetic acid.

Examples of other components that can be incorporated include an antiseptic (ethylparaben, butylparaben, or the like); an antiphlogistic agent (for example, glycyrrhizic acid derivatives, glycyrrhetinic acid derivatives, salicylic acid derivatives, hinokitiol, zinc oxide, and allantoin); a skin whitening agent (for example, a placenta extract, a Saxifraga stolonifera extract, and arbutin); various extracts (for example, phellodendron bark, coptis rhizome, lithospermum root, peony, swertia herb, birch, sage, loquat, carrot, aloe, mallow, iris, grape, coix seed, luffa, lily, saffron, cnidium rhizome, ginger, Hypericum erectum, Ononis spinosa, garlic, capsicum, orange peel, Angelica acutiloba, and sea algae), invigorants (for example, royal jelly, photosensitizers, and cholesterol derivatives); blood circulation accelerators (for example, nonylic acid valenylamide, nicotinic acid benzyl ester, nicotinic acid β-butoxyethyl ester, capsaicin, zingerone, cantharides tincture, ichthammol, tannic acid, α-borneol, tocopherol nicotinate, inositol hexanicotinate, cyclandelate, cinnarizine, tolazoline, acetylcholine, verapamil, cepharanthine, and γ-orizanol); antiseborrheic agents (for example, sulfur, and thianthol); and anti-inflammatory agents (for example, tranexamic acid, thiotaurine, and hypotaurine).

Furthermore, metal sequestering agents such as disodium edetate, trisodium edetate, sodium citrate, sodium polyphosphate, sodium metaphosphate, gluconic acid, and malic acid; caffeine, tannin, verapamil, tranexamic acid and derivatives thereof; various herbal medicine extracts of licorice, Chinese quince, Pyrola japonica and the like; drugs such as tocopherol acetate, glycyrrhetinic acid, glycyrrhizic acid and derivatives thereof, or salts thereof; skin whitening agents such as vitamin C, ascorbic acid, magnesium phosphate, ascorbic acid glucoside, arbutin, and kojic acid; amino acids such as arginine and lysine, and derivatives thereof; sugars such as fructose, mannose, erythritol, threhalose, and xylitol; and the like can also be appropriately incorporated.

There are no particular limitations on the use of the solid powder cosmetic according to the present invention as a product, but the solid powder cosmetic is applicable to solid powder cosmetic in the solid form, such as foundation, eye shadow, cheek color, body powder, perfume powder, baby powder, pressed powder, deodorant powder, and loose powder.

### Method for producing solid powder cosmetic

The solid powder cosmetic of the present invention can be produced by mixing various constituent components including the amide mixture described above, a powder component and an oily component, by a routine method. For example, the solid powder cosmetic can be produced according to any of a method of mixing the constituent components under dry conditions (dry production method), and a method of dispersing the constituent components in a wet system to obtain a slurry, and then drying the slurry to produce the solid powder cosmetic (wet production method).

### Dry production method

When the solid powder cosmetic of the present invention is produced by a dry production method, it is particularly preferable to use a facing rotor type mixing apparatus having a particular structure that will be described below, and to mix the amide mixture described above, a powder component and an oily component. When the facing rotor type mixing apparatus is used, the powder component can be uniformly coated on the surfaces with the oily component without causing aggregation of the powder component. The solid powder cosmetic thus obtainable is excellent in various usage properties such as the feeling of particulate fineness, the moist feeling, smoothness, mealiness, and uniform finish, and also has markedly improved impact resistance.

Here, the facing rotor type mixing apparatus used in the present invention is a mixing apparatus which has a first rotor provided with plural blades and a second rotor provided with plural blades, disposed in a mixing chamber such that the first rotor and the second rotor face each other and respectively have an individual rotating shaft on the same axis line in an approximately horizontal direction, and which mixes raw materials by rotating the first rotor and the second rotor in the same or opposite direction to each other, while supplying the raw materials through a feed port on the first rotor side, and discharges the mixed raw materials through a discharge port on the second rotor side.

When the amide mixture, the powder component and the oily component are mixed using such a facing rotor type mixing apparatus as described above, the powder particles can be uniformly coated on the surfaces with the oily component without causing aggregation of the powder component. Furthermore, since the facing rotor type mixing apparatus used in the present invention is a dry type mixing apparatus, it is not necessary to use the amide mixture, the powder component and the oily component after dissolving them in an appropriate solvent for mixing. Also, the production process is simple and easy as compared with the case of wet mixing, and the facing rotor type mixing apparatus is less problematic in terms of safety or environment.

In addition, the facing rotor type mixing apparatus used in the present invention has been traditionally used as an apparatus for pulverization, and is known to those having ordinary skill in the art as a pulverizing apparatus. For example, the pulverizing apparatuses described in Japanese Unexamined Patent Application No. 2002-79183, Japanese Unexamined Patent Application No. 2003-1127, Japanese Unexamined Patent Application No. 2003-10712, Japanese Unexamined Patent Application No. 2003-71307 and the like can be used as the mixing apparatus of the present invention. Examples of commercially available apparatuses include a cyclone mill (manufactured by Flo-Tec, Ltd.).

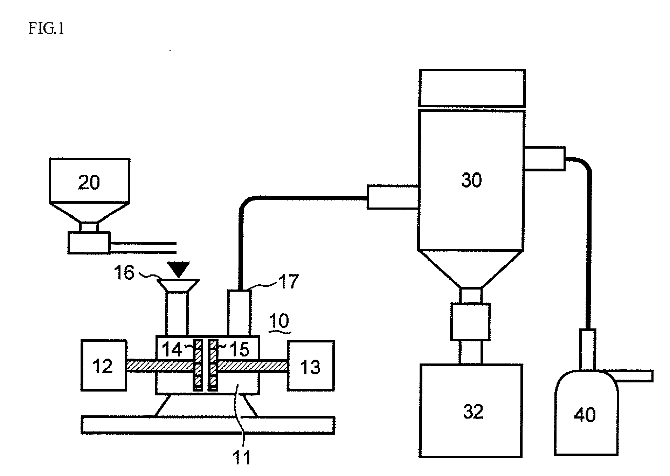

A schematic diagram of one example of the facing rotor type mixing apparatus used in the present invention is presented in Fig. 1. However, the facing rotor type mixing apparatus used in the present invention is not limited to this.

In the facing rotor type mixing apparatus 10, a first rotor 14 and a second rotor 15, which are respectively driven to rotate by motors 12 and 13, are provided inside a mixing chamber 11, in which the first rotor and the second rotor face each other on the same axis line in the horizontal direction. The first rotor 14 and the second rotor 15 are installed to be in communication with a feed port 16 for the raw materials on the first rotor 14 side in the mixing chamber 11, and is in communication with a discharge port 17 on the second rotor 15 side in the mixing chamber 11. Furthermore, a raw material supplying device 20 is provided in the upper part of the feed port 16 of the facing rotor type mixing apparatus 10, and a capturing device 30 (and a collecting container 32) and a suction device 40 are connected to the end of the discharge port 17.

In the facing rotor type mixing apparatus 10, the first rotor 14 and the second rotor 15 that are disposed to face each other on the same axis line in the horizontal direction, rotate integrally with the rotating axes of the motors 12 and 13, respectively. In the facing rotor type mixing apparatus 10, while the first rotor 14 and the second rotor 15 are rotated by the motors 12 and 13 at high speed in the same or in the opposite direction to each other, a raw material mixture to be treated is introduced by the raw material supplying device 20 through the raw material feed port 16. The raw material mixture introduced to the facing rotor type mixing apparatus 10 vigorously collides with the first rotor 14, the second rotor 15, or the inner wall surfaces of the mixing chamber 11, and also, particles of the raw material components collide with each other, so that the raw material components are uniformly mixed and dispersed. As a result, there is obtained a mixture in which the powder particles are uniformly coated on the surfaces with the oily component, without causing aggregation of the powder component.

Furthermore, the first rotor 14 and the second rotor 15 that are facing each other, rotate in the same or opposite direction to each other. Here, in the production method of the present invention, it is suitable to use the first rotor and the second rotor to rotate in the opposite direction. When the rotors are rotated in the opposite direction to each other, a larger shear stress can be generated as compared with the case of rotating the rotors in the same direction. Therefore, a uniform mixture in which aggregation of the powder component does not easily occur, is likely to be obtained. Furthermore, the speed of rotation of the first rotor 14 and the second rotor 15 can be appropriately adjusted to be between, for example, 1000 rpm and 10,000 rpm, and preferably between 3000 rpm and 8000 rpm.

In the first rotor 14 and the second rotor 15, plural blades are provided in a radial direction around the boss provided along the respective rotating shafts of the motors 12 and 13. Usually, the number of blades in one rotor is about 2 to 16 sheets. In the first rotor 14 and the second rotor 15, the shape of the rotor, the number of blades, and the like may be identical with or different from each other.

The subject mixture mixed inside the mixing chamber 11, is discharged through the discharge port 17. Furthermore, the capturing device 30 and the suction device 40 are connected to the end of the discharge port 17. When the suction device 40 is operated, the subject mixture is continuously discharged through the discharge port 17, and the subject mixture thus discharged is captured by the capturing device 30 and is collected inside the collecting container 32. The operating conditions for the suction device 40 can be appropriately adjusted through the type or amount of the subject mixture, the speed of rotation of the rotor, and the like. When the raw material mixture is continuously introduced by the raw material supplying device 20 while the suction device 40 and the capturing device 30 are operated, the mixture can be continuously produced.

The amide mixture, the powder component, and the oily component may be introduced to the facing rotor type mixing apparatus 10 separately or simultaneously. However, in typical cases, it is preferable to carry out preliminary mixing using a simple agitating apparatus such as a Henschel mixer or a Nauta mixer. When the powder component and the oily component are introduced to the facing rotor type mixing apparatus 10 without performing preliminary mixing, there are difficulties in the control of the mixing process, such as that only the light powder component is first discharged without being sufficiently mixed with the oily component.

Furthermore, when the solid powder cosmetic of the present invention is produced by a dry mixing method, usually, a dry mixture of the amide mixture obtained as described above, the powder component and the oily component obtained as described above is filled in, for example, a middle-sized dish container made of a metal or a resin, and is solidified by dry molding. As the method for solidification, conventionally known dry press molding or the like can be used.

### Wet production method

Furthermore, when the solid powder cosmetic of the present invention is produced by a wet production method, the solid powder cosmetic can be produced according to a method of, for example, highly dispersing the raw materials in a wet system using a bead mill, subsequently drying the dispersion product with a flash dryer, and converting the dispersion product into fine particles (W & D production method).

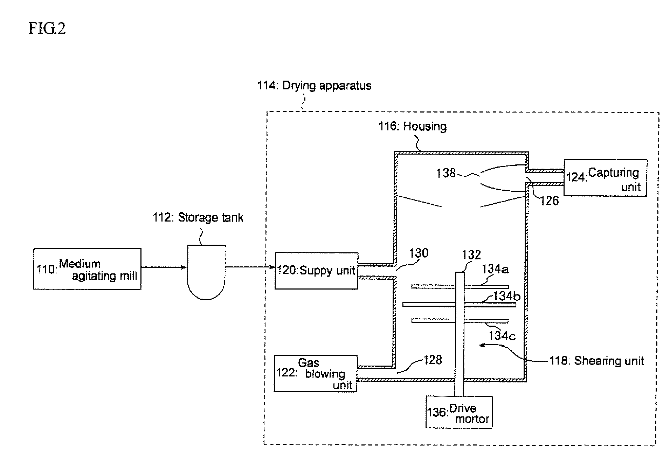

Fig. 2 is a diagram showing an example of the apparatus configuration used in the production method according to an embodiment of the present invention. The method for producing a solid powder cosmetic according to the embodiment of the present invention includes, for example, a slurry preparation step of mixing a powder component, an oily component as a binder, and the above-described amide mixture in a volatile solvent to obtain a slurry; and a drying step of drying the slurry to obtain a dry powder.

First, in the slurry preparation step, a powder component, an oily component, and the above-described amide mixture are mixed in a volatile solvent using a medium agitating mill 110 shown in Fig. 2, and the powder component is cracked/pulverized/dispersed to obtain a slurry. The slurry thus obtained is first collected in a storage tank 112, and is supplied to a drying apparatus 114 at a predetermined flow rate.

The drying apparatus 114 used in the present embodiment converts the slurry into fine liquid droplets using a mechanical shear force, that is, a shear force generated by the rotation of shearing members (plate-shaped members 134a, 134b, and 134c) provided in a shearing unit 118, and blows a dry gas to the fine liquid droplets to perform drying of the slurry. There are no particular limitations on the shape of the shearing members as long as the shape is adequate for the purpose of the present invention. The shape of the shearing members may be any shape, for example, a blade shape or a disc shape, in addition to the plate shape such as described above.

As such, in the present embodiment, since a dry powder is produced using a drying apparatus 114 which performs drying while the slurry is in the form of fine liquid droplets, a dry powder in which aggregation of the powder component almost does not occur at the time of drying, can be obtained. For that reason, it is possible to provide a solid powder cosmetic which has an excellent feeling of use when applied on the skin. Also, since it is not necessary to carry out cracking again after drying, the method is excellent in terms of productivity and the work environment.

Furthermore, it is also suitable to further include a step of filling the dry powder thus obtained in a container, and solidifying the dry powder by dry press molding. The solid powder cosmetic thus obtained is excellent not only in the feeling of use, but also in the usage properties such as appliability with a puff.

As shown in Fig. 2, it is suitable to use a medium agitating mill 110 in the slurry preparation step, in order to mix the powder component and the oily component as a binder in a volatile solvent. When a medium agitating mill is used, a slurry in which the surfaces of the powder component is cleanly coated with the oily component can be obtained, and when this slurry is used, a solid powder cosmetic having a superior feeling of use and superior usage properties can be obtained.

### <Slurry preparation step>

As the method of mixing a powder component and an oily component in a volatile solvent to obtain a slurry, methods such as described below may be used.
(A) A method of adding a product which has been prepared by dry mixing/cracking a powder and an oil component in advance with a Henschel mixer (registered trademark), a pulverizer or the like, into a volatile solvent, and mixing/dispersing the components using a disper-mixer, a homogenizer, a planetary mixer, a twin-screw kneading machine or the like.
(B) A method of adding a powder and an oil component into a volatile solvent, preliminarily mixing the components with a disper-mixer or the like as necessary, and then subjecting the mixture to cracking/pulverization/dispersion treatments using a medium agitating mill.
(C) A method of adding a certain specific powder component having strong cohesiveness, such as an elastic polymer powder or a fine particle powder, into a volatile solvent, preliminarily mixing this powder component with the solvent with a disper-mixer as necessary, subsequently cracking/pulverizing/dispersing the mixture using a medium agitating mill to obtain a dispersion liquid, adding another powder component and an oil component to the dispersion liquid, and further treating the mixture using a wet mixing machine or a medium agitating mill.

In addition, it is suitable to use a medium agitating mill in the slurry preparation step (for example, the above-described methods (B) and (C)). A medium agitating mill is a device which accommodates a dispersion liquid formed from a powder component (and an oily component) and a solvent in a vessel containing a solid dispersing medium (medium) such as beads, and agitating the liquid in the vessel to perform cracking/pulverization/dispersion of the powder component by means of an impact force, a frictional force or the like exerted by the medium.

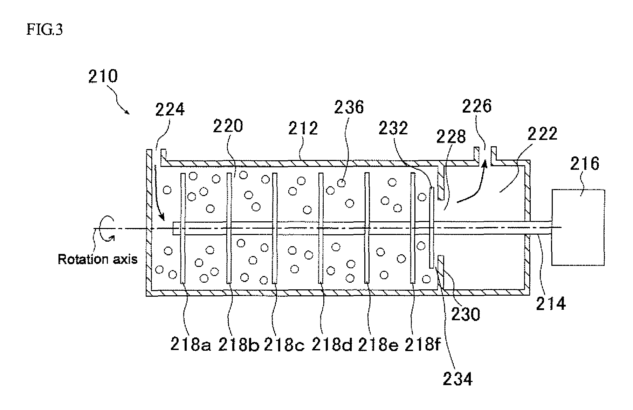

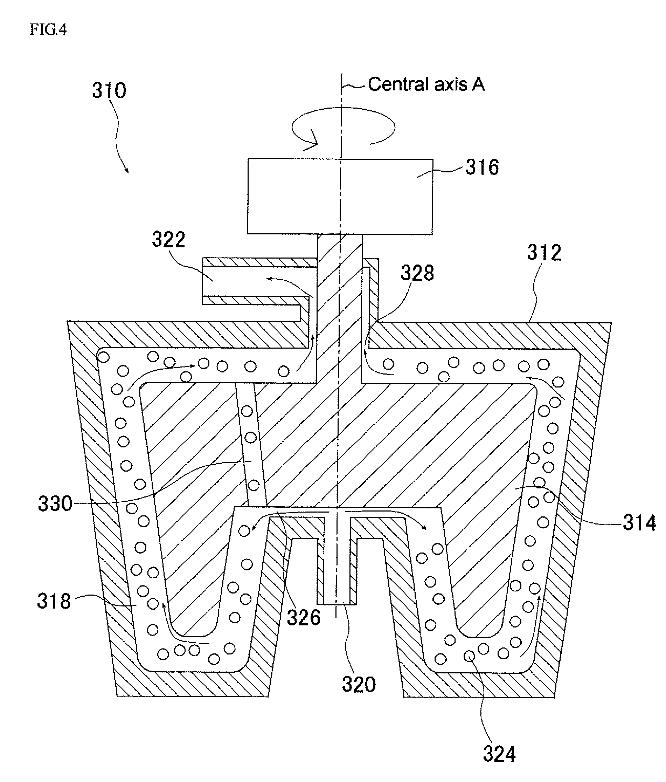

Fig. 3 and Fig. 4 are schematic configuration diagrams showing examples of the medium agitating mill that is suitably used in the present invention. The medium agitating mill that can be suitably used in the present invention is not particularly limited to the following examples, and any medium agitating mill that can achieve the purpose of the present invention may be used.

The medium agitating mill 210 of the example shown in Fig. 3 includes an approximately cylindrical-shaped vessel 212, a drive shaft 214 inserted across the vessel 212, a drive motor 216 which rotationally drives the drive shaft 214, and plural sheets of agitating discs 218a to 218f that are fitted to the drive shaft 214. The inside of the vessel 212 is divided into a dispersing chamber 220 which performs cracking/pulverization/dispersion of the powder component, and a discharge chamber 222 which discharges the treated dispersion liquid. The dispersing chamber 220 of the vessel 212 is provided with a supply port 224 for supplying a dispersion liquid to be treated, and the discharge chamber 222 is provided with a discharge port 226 for removing the treated dispersion liquid. A partition wall 230 provided with an opening 228 is provided between the dispersing chamber 220 and the discharge chamber 222, and near this partition wall 230, a separating disc 232 fitted to the drive shaft 214 is disposed to cover the opening 228 of the partition wall 230. A gap is provided between the partition wall 230 and the separating disc 232, and this gap is used as a separating slit 234 which separates the solid dispersing medium from the dispersion liquid to be treated.

The dispersion liquid containing a powder component and a solvent is successively supplied through the supply port 224 to the dispersing chamber 220 inside the vessel 212, and the dispersion liquid in the dispersing chamber 220 moves successively in the direction of the discharge chamber 222. At this time, the drive shaft 214 is rotationally driven by the drive motor 216, and the agitating discs 218a to 218f are rotating. The dispersing chamber 220 is filled with a large number of solid dispersing media 236, and the solid dispersing media 236 are agitated together with the dispersion liquid by the rotation of the agitating discs 218a to 218f. The aggregated powder component in the dispersion liquid is cracked/pulverized/dispersed by the impact force, shear stress or the like exerted by the solid dispersing media 236.

The dispersion liquid that has been subjected to cracking/pulverization/dispersion as described above, passes through the separating slit 234 that is located between the partition wall 230 between the dispersing chamber 220 and the discharge chamber 222, and the separating disc 232, flows into the discharge chamber 222 and is discharged to the outside through the discharge port 226. The separating slit 234 has a size that will prevent the solid dispersing media 236 from escaping from the dispersing chamber 220 to the discharge chamber 222. Accordingly, when the dispersion liquid passes through the separating slit 234, the separating slit separates the dispersion liquid (powder component + solvent) from the solid dispersing media 236, so that only the dispersion liquid enters the discharge chamber.

Fig. 4 is a schematic configuration diagram of an annular type medium agitating mill. The medium agitating mill 310 of Fig. 4 includes a vessel 312 which has an approximately W-shaped cross-section that is symmetric about the central axis A, a rotor 314 which is provided on the vessel 312, has an approximately inverse U-shape and is capable of rotating around the central axis A, and a drive motor 316 that rotationally drives the rotor 314. A circular space 318 is formed between the inner surface of the vessel 316 and the outer surface of the rotor 314, and this circular space 318 has a shape having an approximately V-shaped cross-section which lies on both sides of the central axis A. Furthermore, a supply port 320 for sending the dispersion liquid (powder component + solvent) to be treated to the circular space 318, and a discharge port 322 for removing the dispersion liquid to be treated from the circular space 318, are formed in the vessel 312. The circular space 318 is filled with solid dispersing media 324, so that the circular space 218 is used as a dispersing chamber that performs cracking/pulverization/dispersion of the powder component in the dispersion liquid.

The dispersion liquid supplied from the supply port 320 is transported to the circular space 318 via an inlet slit 326. The dispersion liquid thus transported moves inside the circular space 318 and is discharged through the discharge port 322 via an outlet slit 328. At this time, the dispersion liquid and the solid dispersing media 324 inside the circular space 318 are agitated by rotating the rotor 314 about the central axis A within the circular space 318. Then, the aggregated powder component in the dispersion liquid is cracked/pulverized/dispersed by the impact force, shear stress or the like exerted from the solid dispersing media 324. Thereafter, the dispersion liquid passes through the outlet slit 328 and is removed through the discharge port 322.

The outlet slit 328 has a size that will prevent the solid dispersing media 324 from escaping from the circular space 318, and functions as a separating unit that separates the dispersion liquid (powder component + solvent) from the solid dispersing media 324. Furthermore, the rotor 314 is provided with a returning hole 330 for returning the solid dispersing media 324 to the inlet side, so that the solid dispersing media 324 are kept from staying in the vicinity of the outlet.

The reason for cracking/pulverizing/dispersing a powder and an oil component in a volatile solvent using a medium agitating mill is that since the mixed and dispersed state of the powder component and the oily component can be improved, and the surface of the powder component can be uniformly coated with the oily component, a solid powder cosmetic having a good feeling of use can be obtained. Furthermore, a powder having strong cohesiveness can be easily cracked and uniformly dispersed in a volatile solvent.

Examples of the medium agitating mill have been described above, but in addition to those, suitable examples include batch type bead mills such as a basket mill; horizontal type, vertical type, and annular type continuous bead mills; sand grinder mills, ball mills, and Micros (registered trademark). However, there are no particular limitations on the medium agitating mill as long as the medium agitating mill conforms to the purpose of the present invention. That is, any medium agitating mill which, when a powder component that is in an aggregated state is incorporated, can crack the aggregation of the powder component and disperse with agitation the powder component to a state close to primary particles to thereby uniformly attach the oily component on the surfaces of the powder, can be used without any particular limitations.

The media to be used in the medium agitating mill are preferably beads, and beads produced from raw materials such as glass, alumina, zirconia, steel and flintstone can be used. Particularly, beads made of zirconia are preferred. In regard to the size of the beads, usually beads having a diameter of about 0.5 to 10 mm are used with preference, but in the present invention, beads having a diameter of approximately 2 mm to 5 mm are used with preference. If the size of the bead diameter is too small, cracking of the extender pigments such as mica and talc proceeds excessively, and an adverse effect is exerted on the feeling of use, or the hardness after molding is increased. As a result, appliability deteriorates, and caking or the like is easily induced. On the other hand, if the size of the bead is too large, aggregation of the powder component cannot be sufficiently cracked, and uniform coating with the oily component becomes difficult.

There are no particular limitations on the volatile solvent that is used in the present invention, but examples of the volatile solvent include purified water, cyclic silicones, ethanol, light liquid isoparaffin, lower alcohols, ethers, LPG, fluorocarbons, N-methylpyrrolidone, fluoroalcohols, volatile linear silicones, and next generation Freon. These solvents are used properly with different purposes, singly or as mixtures of two or more kinds, in accordance with the characteristics of the powder component or the characteristics of the oily component used.

The amount ratio (mass ratio) of the powder component and the oily component in the slurry preparation step depends on the type of the oily component and the powder component used, but the amount ratio is suitably such that powder component/oily component = 60/40 to 99.5/0.5. At this time, since the amount of the volatile solvent used depends on the polarity, specific gravity and the like of the volatile solvent used, the amount of the volatile solvent cannot be specifically defined. However, it is important to secure the fluidity that enables the treatment with a medium agitating mill.

### <Drying step>

Next, an example of the drying apparatus that is used in the drying step of an embodiment according to the present invention will be described with reference to Fig. 2. The drying apparatus used in the production method according to the present embodiment is not limited to the apparatus of Fig. 2, and any drying apparatus equipped with a shearing unit which mechanically converts a slurry into fine liquid droplets is acceptable. The drying apparatus 14 shown in Fig. 2 includes a hollow-shaped housing 16 which serves as the site for performing drying of the slurry; a shearing unit 18 which converts a slurry into fine liquid droplets by means of rotating shearing members (plate-shaped members 34a, 34b, and 34c) that are provided inside the housing 16; a supply unit 20 which supplies the slurry to the shearing members (plate-shaped members 34a, 34b and 34c) inside the housing 16; a gas blowing unit 22 which blows a dry gas into the housing 16, and supplies the dry gas to the slurry that has been converted to fine liquid droplets by the shearing unit 18; and a capture unit 24 which captures a powdery composition generated by drying the slurry.

The housing 16 has an approximately cylindrical shape which is vertical and hollow, and is provided at the upper part with an emission port 26 which emits the powdery composition and the dry gas, and is provided at the lower part with a blowing port 28 which supplies the dry gas from the gas blowing unit 22 into the housing 16. Also, a supply port 30 which supplies the slurry into the housing 16 is disposed between the emission port 26 located at the upper part of the housing 16 and the blowing port 28 located at the lower part.

The shearing unit 18 includes a rotating shaft 32 that is installed in the direction perpendicular to the bottom of the housing 16, shearing members (plate-shaped members 34a, 34b and 34c) that are provided at a right angle to the rotating shaft 32, and a drive unit 36 for rotating the rotating shaft 32. The drive unit 36 is disposed outside the housing 16, and transfers a rotating force to the shearing members (plate-shaped members 34a, 34b and 34c) through the rotating shaft 32. The shearing members shown in Fig. 1 consist of three plate-shaped members 34a, 34b and 34c that are installed perpendicularly to the rotating shaft 32 at regular intervals in the vertical direction. These shearing members are located at the lower part of the supply port 30 for the slurry and in the upper part of the blowing port 28 for the dry gas. When the rotating shaft 32 is rotated by the drive unit 36 composed of a motor an the like, the plate-shaped members 34a, 34b and 34c rotate in the horizontal direction about the rotating shaft 32 inside the housing 16, and this mechanical shear force causes the conversion of the slurry into fine liquid droplets.

The supply unit 20 supplies the slurry sent from the storage tank 12 into the housing 16. The slurry supplied to the housing 16 drops toward the plate-shaped members 34a, 34b and 34c, and is converted into fine liquid droplets by the rotating plate-shaped members 34a, 34b and 34c. Furthermore, the dry gas sent from the gas blowing unit 22 is blown into the housing 16 through the blowing port 28. The dry gas is supplied toward the tangential direction of the horizontal cross-section of the housing 16, and since the plate-shaped members 34a, 34b and 34c are in a rotating movement, the dry gas stream blown into the housing 16 is changed to a swirling flow. When the slurry in the form of fine liquid droplets is brought into contact with this dry gas stream, the slurry is further micronized and is dried to form a powdery composition. This powdery composition is blown up together with the dry gas stream to the upper part of the housing 16, and is emitted through the emission port 26. The powdery composition emitted out of the housing 16 through the emission port 26 is captured by a capture unit 24.

Furthermore, a classification unit 38 is provided in the area of the emission port 26 inside the housing 16. The classification unit 38 is constructed as an orifice provided at the emission port 26, and prevents large grains, lumps, undried materials and the like from entering the collection unit 24. The configuration of the classification unit is not limited to this, and any other configuration may be used.

As such, when a mechanical shear force is exerted by the shearing members (plate-shaped members 34a, 34b and 34c), and the slurry is converted to fine liquid droplets and dried, a powdery composition with less aggregation can be obtained. The reason why a powdery composition with less aggregation is obtained is speculated to be that when the slurry is converted to fine liquid droplets, the amount of the powder component present in the liquid droplets is small, and therefore, aggregation at the time of drying does not easily occur, and that the aggregation of the powder component occurring during the drying process is cracked by the shear force exerted by the shearing members or the swirling flow.

An example of using shearing members consisting of plate-shaped members that rotate in the horizontal direction has been described herein, but in addition to this, shearing members consisting of plate-shaped members that rotate in the vertical direction (the rotating shaft extending in the horizontal direction) may also be provided. Furthermore, the shape of the shearing member is not limited to the shape described above, and for example, a blade shape (a cutter provided perpendicularly to the tip of a rod-shaped member which is attached perpendicularly to the rotating shaft), a disc shape and the like may be used. Also, there are no particular limitations on the number of the shearing members, or the like.

The drying apparatus described above is of a type called flash dryer, and examples thereof include a Spin Flash Dryer manufactured by APV Nordic Anhydro A/S, a DryMeister manufactured by Hosokawa Micron Corp., and a vertical agitation dryer manufactured by Tsukishima Kikai Co., Ltd. There are no limitations on the drying apparatus that may be suitably used in the present invention, and any of vertical type and horizontal type drying apparatuses may be used as long as the apparatus has a shearing mechanism in the system.

The temperature of the dry gas used at the time of drying can be varied depending on the boiling point of the volatile solvent used. Also, since the drying efficiency increases as the temperature of the dry gas is higher, it is desirable to set the temperature of the dry gas high, to the extent that there are no adverse effects such as heat-induced alteration of the dry powder constituent components.

Furthermore, when an inert gas such as nitrogen gas or Ar gas is included in the housing 16, the apparatus acquires excellent explosion proofness, and therefore, the work environment properties are also improved. In addition, recovery of the solvent is also made possible by adopting a solvent recovery mechanism such as a condenser.

### <Solidification step>

In regard to the method for producing a solid powder cosmetic according to the embodiment of the present invention, it is suitable that the production method further include a solidification step of filling the dry powder in a container and solidifying the dry powder by dry molding. As the method for solidification, conventionally known dry press molding and the like may be used. A solid powder cosmetic obtained as such maintains an excellent feeling of use, which is an advantage of solid powder cosmetic produced by wet methods, and also has good usage properties (appliability with a puff), which is an advantage of dry molding. Furthermore, in the case of conventional wet molding methods including a step of filling a slurry in a container by injection filling, since it is necessary to take into consideration of the fillability of the slurry, there are limitations in the raw materials to be used. However, it is an advantage of the production method of the present invention that there are no limitations on the raw materials to be used, as long as conventional dry press molding is carried out.

The amount of incorporation of the dry powder upon obtaining a solid powder cosmetic preferably 0.5 to 100 parts by mass, and more preferably 30 to 100 parts by mass, relative to 100 parts by mass of the cosmetic.

In the case of producing a solid powder cosmetic containing an added pearl pigment, representative examples of which include titanated mica and glass pearl, it is preferable to first obtain a dry powder using the portion of the powder component excluding the pearl pigment, by subjecting the powder component to the slurry preparation step and the drying step described above. This dry powder and a needed amount of a pearl pigment are mixed in a dry mixing machine which exerts a weak shear force, such as a Henschel mixer or a Nauta mixer, to obtain a mixed powder, and this mixed powder is filled in a container or is further subjected to dry molding. Thus, a solid powder cosmetic is obtained. The solid powder cosmetic obtained by this method is excellent in terms of the feeling of use and usage properties, and also has an excellent pearl-like texture.

### EXAMPLES

Hereinafter, the present invention will be described in more detail by way of Examples, but the present invention is not intended to be limited thereto. The amounts in the following formulations are expressed in percent (%) by mass.

### <Evaluation of usage properties of solid powder cosmetic >

### (A) Evaluation of long-lasting makeup effect (makeup running and oily shining)

Foundations having the formulations as indicated in the following table were prepared by the slurry preparation process and the solid powder cosmetic production process described above, and the long-lasting makeup effect of the foundations was subjected to a panel test by a method described below. A panel of ten cosmetic experts applied each of the obtained foundations on the skin. After 3 hours, three expert evaluators performed a 10-grade evaluation (very poor long-lasting makeup effect: 0 point - very good long-lasting makeup effect: 10 points) in terms of the respective evaluation items of "makeup running evaluation" and "oily shining evaluation" according to the following evaluation criteria. The evaluation results were judged from the average points of the three expert evaluators according to the following judgment criteria.

### [Judgment]

A: The average point of the evaluation points is equal to or greater than 9 and less than 10.
B: The average point of the evaluation points is equal to or greater than 6 and less than 8.
C: The average point of the evaluation points is equal to or greater than 4 and less than 5.
D: The average point of the evaluation points is equal to or greater than 2 and less than 3.
E: The average point of the evaluation points is less than 2.

### (B) Evaluation of usage properties (easy appliability)

A panel of ten cosmetic experts applied each of the obtained foundations on the skin, and performed a 5-grade evaluation (very difficult to apply: 0 point - very easy to apply: 5 points) on the evaluation item of "easy applicability", with respect to the differences before and after the treatment.

### (C) Evaluation of water repellency and oil repellency

In the evaluation of water repellency, an observation was made by applying a produced powder on a surface coated with a water-insoluble composite film, and dropping 20 mg of water droplets thereon. Furthermore, in the evaluation of oily repellency, an observation was made by applying a produced powder on a surface coated with an oil-insoluble composite, and dropping 20 mg of oleic acid or squalene. The results of the observations were subjected to a 5-grade evaluation (significantly wetted: 0 point - very repellent: 5 points).

First, foundations to which the particular amide mixture of the present invention was incorporated as indicated in the following Table 1, were prepared, and an evaluation of the foundations was carried out.

**[Table 1]**

| | Production Example 1-1 | Production Example 1-2 | Production Example 1-3 | Production Example 1-4 | Production Example 1-5 | Production Example 1-6 | Production Example 1-7 |
|---|---|---|---|---|---|---|---|
| Silicone-treated talc | 23 | 23 | 23 | 23 | 23 | 23 | 23 |
| Silicone-treated sericite | Balance | Balance | Balance | Balance | Balance | Balance | Balance |
| Mica | 15 | 15 | 15 | 15 | 15 | 15 | 15 |
| Plate-shaped barium sulfate | 5 | 5 | 5 | 5 | 5 | 5 | 5 |
| Spherical silicone powder | 10 | 10 | 10 | 10 | 10 | 10 | 10 |
| Alkylsilicone-treated iron oxide | 5 | 5 | 5 | 5 | 5 | 5 | 5 |
| Titanium oxide fine particles | 5 | 5 | 5 | 5 | 5 | 5 | 5 |
| Antiseptic | 0.2 | 0.2 | 0.2 | 0.2 | 0.2 | 0.2 | 0.2 |
| Silicone-treated titanium oxide | 10 | 10 | 10 | 10 | 10 | 10 | 10 |
| Amide mixture *1 | - | 1 | 2 | 5 | 10 | 15 | 20 |
| Diphenylsiloxyphenyltrimethicone | 2 | 2 | 2 | 2 | 2 | 2 | 2 |
| Diisostearyl malate | 3 | 3 | 3 | 3 | 3 | 3 | 3 |
| Octyl methoxycinnamate | 3 | 3 | 3 | 3 | 3 | 3 | 3 |
| Sorbitan sesquiisostearate | 1 | 1 | 1 | 1 | 1 | 1 | 1 |
| Evaluation of makeup running | E | C | B | B | B | B | B |
| Evaluation of oily shining | E | C | C | C | c | c | C |
| Evaluation of easy appliability | 5 | 5 | 4 | 4 | 4 | 3 | 0 |

| | | | | | | | |
|---|---|---|---|---|---|---|---|
| *1: An amide mixture obtained using a mixture of hexamethylenediamine and bisaminomethylcyclohexane, and hydrogenated castor oil fatty acids, by performing an amidation reaction by a conventional method | | | | | | | |

(Production method) The various components indicated in the formulations of the above Table 1 were dry mixed and pulverized using a pulverizer, and then the mixture was press molded. Thus, a solid-state solid powder cosmetic was obtained.

As shown in the above Table 1, in regard to the Production Examples 1-2 to 1-7 in which an amide mixture obtained by amidating a mixture of hexamethylenediamine and bisaminomethylcyclohexane with hydrogenated castor oil fatty acids is incorporated into a solid powder cosmetic in an amount of 1% to 20% by mass, relatively satisfactory results were obtained in all cases in the evaluations of makeup running, oily shining, and easy appliability. Particularly, the Production Examples 1-3 to 1-5 in which the amide mixture was incorporated in an amount of 2% to 10% by mass, were considered excellent in the evaluation of makeup running.

On the contrary, in regard to the Production Example 1-1 in which the amide mixture was not incorporated, the results for the evaluation of easy appliability were satisfactory, but the results for the evaluations of makeup running and oily shining were very poor. As the amount of incorporation of the amide mixture increased, the results for the evaluation of easy appliability tended to become poorer. In the Production Example 1-7 in which the amide mixture was incorporated in an amount of 20% by mass, the foundation was very difficult to apply.

Subsequently, foundations in which a spherical porous polymethyl methacrylate resin powder was further incorporated together with the particular amide mixture of the present invention as indicated in the following Table 2, were prepared, and an evaluation of the foundations was carried out.

**[Table 2]**

| | Production Example 2-1 | Production Example 2-2 | Production Example 2-3 | Production Example 2-4 | Production Example 2-5 | Production Example 2-6 | Production Example 2-7 |
|---|---|---|---|---|---|---|---|
| Silicone-treated talc | 23 | 23 | 23 | 23 | 23 | 23 | 23 |
| Silicone-treated sericite | Balance | Balance | Balance | Balance | Balance | Balance | Balance |
| Mica | 15 | 15 | 15 | 15 | 15 | 15 | 15 |
| Plate-shaped barium sulfate | 5 | 5 | 5 | 5 | 5 | 5 | 5 |
| Spherical porous PMMA resin powder *1 | - | 1 | 2 | 5 | 10 | 15 | 20 |
| Alkylsilicone-treated iron oxide | 5 | 5 | 5 | 5 | 5 | 5 | 5 |
| Titanium oxide fine particles | 5 | 5 | 5 | 5 | 5 | 5 | 5 |
| Antiseptic | 0.2 | 0.2 | 0.2 | 0.2 | 0.2 | 0.2 | 0.2 |
| Silicone-treated titanium oxide | 10 | 10 | 10 | 10 | 10 | 10 | 10 |
| Amide mixture *2 | 5 | 5 | 5 | 5 | 5 | 5 | 5 |
| Diphenylsiloxyphenyltrimethicone | 2 | 2 | 2 | 2 | 2 | 2 | 2 |
| Diisostearyl malate | 3 | 3 | 3 | 3 | 3 | 3 | 3 |
| Octyl methoxycinnamate | 3 | 3 | 3 | 3 | 3 | 3 | 3 |
| Sorbitan sesquiisostearate | 1 | 1 | 1 | 1 | 1 | 1 | 1 |
| Evaluation of makeup running | B | B | B | B | B | B | B |
| Evaluation of oily shining | C | B | B | A | A | A | A |
| Evaluation of easy appliability | 4 | 4 | 4 | 4 | 4 | 3 | 1 |

| | | | | | | | |
|---|---|---|---|---|---|---|---|
| *1: TECHPOLYMER MBP-8HP: manufactured by Sekisui Chemical Co., Ltd. (average particle size 8 µm, specific surface area 150 m²/g, and most frequent pore diameter 180 Å) *2: An amide mixture obtained using a mixture of hexamethylenediamine and bisaminomethylcyclohexane, and hydrogenated castor oil fatty acids, by performing an amidation reaction by a conventional method | | | | | | | |

(Production method) The various components indicated in the formulations of the above Table 2 were dry mixed and pulverized using a pulverizer, and then the mixture was press molded. Thus, a solid-state solid powder cosmetic was obtained.

As shown in the Table 2, it was found that in the Production Examples 2-2 to 2-7 in which a spherical porous polymethyl methacrylate powder was further incorporated in an amount of 1% to 20% by mass in addition to a certain amount of the amide mixture, the results for the evaluation of oily shining were improved. Particularly, in the Production Examples 2-3 to 2-6 in which the spherical porous PMMA resin powder was incorporated in an amount of 2% to 15% by mass, very excellent results were obtained for the evaluation of oily shining.

Next, foundations in which an organically modified clay mineral was further incorporated together with the particular amide mixture of the present invention as indicated in the following Formulation Table 3, were prepared, and an evaluation of the foundations was carried out.

**[Table 3]**

| | Production Example 3-1 | Production Example 3-2 | Production Example 3-3 | Production Example 3-4 | Production Example 3-5 |
|---|---|---|---|---|---|
| Silicone-treated talc | 23 | 23 | 23 | 23 | 23 |
| Silicone-treated sericite | Balance | Balance | Balance | Balance | Balance |
| Mica | 15 | 15 | 15 | 15 | 15 |
| Plate-shaped barium sulfate | 5 | 5 | 5 | 5 | 5 |
| Spherical silicone powder | 10 | 10 | 10 | 10 | 10 |
| Alkylsilicone-treated iron oxide | 5 | 5 | 5 | 5 | 5 |
| Titanium oxide fine particles | 5 | 5 | 5 | 5 | 5 |
| Antiseptic | 0.2 | 0.2 | 0.2 | 0.2 | 0.2 |
| Silicone-treated titanium oxide | 10 | 10 | 10 | 10 | 10 |
| Amide mixture *1 | - | 0.5 | 2.5 | 7.5 | 10 |
| Organically modified clay mineral *2 | - | 0.5 | 2.5 | 7.5 | 10 |
| Diphenylsiloxyphenyltrimethicone | 2 | 2 | 2 | 2 | 2 |
| Diisostearyl malate | 3 | 3 | 3 | 3 | 3 |
| Octyl methoxycinnamate | 3 | 3 | 3 | 3 | 3 |
| Sorbitan sesquiisostearate | 1 | 1 | 1 | 1 | 1 |
| Evaluation of makeup running | E | B | A | A | A |
| Evaluation of oily shining | E | B | B | B | B |
| Evaluation of easy appliability | 5 | 4 | 4 | 3 | 0 |

| | | | | | |
|---|---|---|---|---|---|
| *1: An amide mixture obtained using a mixture of hexamethylenediamine and bisaminomethylcyclohexane, and hydrogenated castor oil fatty acids, by performing an amidation reaction by a conventional method *2: Bentone 38 (manufactured by National Lead Company) | | | | | |

(Production method) The various components indicated in the formulations of the above Table 3 were dry mixed and pulverized using a pulverizer, and then the mixture was press molded. Thus, a solid-state solid powder cosmetic was obtained.

As shown in the Table 3, it was confirmed that when the amide mixture and an organically modified clay mineral were used in combination, the results for the evaluations of makeup running and oily shining were markedly improved. Particularly, in the Examples 3-2 to 3-4 in which the amide mixture and an organically modified clay mineral were incorporated together in an amount of 1% to 15% by mass, satisfactory results were obtained in all cases in the evaluations of makeup running and oily shining as well as in the evaluation of easy appliability. On the contrary, the Production Example 3-1 in which the amide mixture and the organically modified clay mineral were not incorporated, was poor in the evaluations of makeup running and oily shining. Furthermore, the Production Example 3-5 in which the amide mixture and the organically modified clay mineral were incorporated together in an amount of 20% by mass, was not satisfactory in terms of easy appliability.

In addition, foundations in which the particular amide mixture of the present invention and an organically modified clay mineral were incorporated, and a spherical porous polymethyl methacrylate resin powder was further incorporated as indicated in the following Formulation Table 4, were prepared, and an evaluation of the foundations was carried out.

**[Table 4]**

| | Production Example 4-1 | Production Example 4-2 | Production Example 4-3 | Production Example 4-4 | Production Example 4-5 |
|---|---|---|---|---|---|
| Silicone-treated talc | 23 | 23 | 23 | 23 | 23 |
| Silicone-treated sericite | Balance | Balance | Balance | Balance | Balance |
| Mica | 15 | 15 | 15 | 15 | 15 |
| Plate-shaped barium sulfate | 5 | 5 | 5 | 5 | 5 |
| Spherical silicone powder | 10 | - | - | - | - |
| Spherical porous PMMA resin powder *1 | - | 5 | 10 | 20 | 30 |
| Alkylsilicone-treated iron oxide | 5 | 5 | 5 | 5 | 5 |
| Titanium oxide fine particles | 5 | 5 | 5 | 5 | 5 |
| Antiseptic | 0.2 | 0.2 | 0.2 | 0.2 | 0.2 |
| Silicone-treated titanium oxide | 10 | 10 | 10 | 10 | 10 |
| Amide mixture *2 | 2.5 | 2.5 | 2.5 | 2.5 | 2.5 |
| Organically modified clay mineral *3 | 2.5 | 2.5 | 2.5 | 2.5 | 2.5 |
| Diphenylsiloxyphenyltrimethicone | 2 | 2 | 2 | 2 | 2 |
| Diisostearyl malate | 3 | 3 | 3 | 3 | 3 |
| Octyl methoxycinnamate | 3 | 3 | 3 | 3 | 3 |
| Sorbitan sesquiisostearate | 1 | 1 | 1 | 1 | 1 |
| Evaluation of makeup running | A | A | A | A | A |
| Evaluation of oily shining | B | A | A | A | A |
| Evaluation of easy applicability | 4 | 5 | 5 | 4 | 1 |

| | | | | | |
|---|---|---|---|---|---|
| *1: TECHPOLYMER MBP-8HP: manutactured by Sekisui Chemical Co., Ltd. (average particle size 8 µm, specific surface area 150 m²/g, and most frequent pore diameter 180 Å) *2: An amide mixture obtained using a mixture of hexamethylenediamine and bisaminomethylcyclohexane, and hydrogenated castor oil fatty acids, by performing an amidation reaction by a conventional method *3: Bentone 38 (manufactured by National Lead Company) | | | | | |

(Production method) The various components indicated in the formulations of the above Table 4 were dry mixed and pulverized using a pulverizer, and then the mixture was press molded. Thus, a solid-state solid powder cosmetic was obtained.

As shown in the Table 4, it was confirmed that when the amide mixture and an organically modified clay mineral were added, and a spherical porous polymethyl methacrylate resin powder was further incorporated in an amount of 1% to 20% by mass, the results for the evaluations of makeup running and oily shining, and the results for the evaluation of easy appliability were further improved (Production Examples 4-2 to 4-4). On the other hand, the Production Example 4-5 in which the spherical porous PMMA resin powder was incorporated in an amount of 30% by mass, exhibited excellent results for the evaluation of oily shining, but exhibited poor results for the evaluation of easy appliability.

Next, foundations in which the surfaces of the powder component were treated with a fluorine compound by further adding a fluorine compound at the time of mixing the powder component and the oily component as indicated in the following Formulation Table 5, were prepared, and an evaluation of the foundations was carried out.

**[Table 5]**

| | Production Example 5-1 | Production Example 5-2 | Production Example 5-3 | Production Example 5-4 | Production Example 5-5 | Production Example 5-6 |
|---|---|---|---|---|---|---|
| Silicone-treated talc | 23 | 23 | 23 | 23 | 23 | 23 |
| Silicone-treated sericite | Balance | Balance | Balance | Balance | Balance | Balance |
| Mica | 15 | 15 | 15 | 15 | 15 | 15 |
| Plate-shaped barium sulfate | 5 | 5 | 5 | 5 | 5 | 5 |
| Spherical silicone powder | 10 | 10 | 10 | 10 | 10 | 10 |
| Alkylsilicone-treated iron oxide | 5 | 5 | 5 | 5 | 5 | 5 |
| Titanium oxide fine particles | 5 | 5 | 5 | 5 | 5 | 5 |
| Antiseptic | 0.2 | 0.2 | 0.2 | 0.2 | 0.2 | 0.2 |
| Silicone-treated titanium oxide | 10 | 10 | 10 | 10 | 10 | 10 |
| Amide mixture *1 | 5 | 5 | 5 | 5 | 5 | 5 |
| Diphenylsiloxyphenyltrimethicone | 2 | 2 | 2 | 2 | 2 | 2 |
| Diisostearyl malate | 3 | 3 | 3 | 3 | 3 | 3 |
| Octyl methoxycinnamate | 3 | 3 | 3 | 3 | 3 | 3 |
| Sorbitan sesquiisostearate | 1 | 1 | 1 | 1 | 1 | 1 |
| Fluorine compound *2 | - | 0.1 | 1 | 5 | 10 | 15 |
| Water repellency | 3 | 4 | 5 | 5 | 5 | 5 |
| Oil repellency | 3 | 4 | 5 | 5 | 5 | 5 |
| Evaluation of easy appliability | 4 | 4 | 4 | 4 | 3 | 1 |

| | | | | | | |
|---|---|---|---|---|---|---|
| *1: An amide mixture obtained using a mixture of hexamethylenediamine and bisaminomethylcyclohexane, and hydrogenated castor oil fatty acids, by performing an amidation reaction by a conventional method *2: FOMBLIN HC/P2-1000; manufactured by Solvay Solexis SPA (fluorine compound represented by the following formula (IV)) | | | | | | |

X-CF₂O-(CF₂CF₂O)p-(CF₂O)q-CF₂-X (IV)

wherein in the formula (IV), X represents CH₂C-(OCH₂CH₂)p-OPO(OH)₂; and the ratio of p/q is 0.5 to 3.0.

### (Production method)

The various components indicated in the above Formulation Table 5 were dispersed and mixed in ethyl alcohol with a disper-mixer, and the slurry viscosity was adjusted to about 2000 mPa·s. Subsequently, the slurry was subjected to cracking/pulverization/dispersion using a medium agitating mill (sand grinder mill) filled with 2-mmφ zirconia beads, and thus a powder slurry was obtained. The powder slurry was dried in the form of fine liquid droplets using an agitating drying apparatus, DryMeister (manufactured by Hosokawa Micron Group), and ethyl alcohol was vaporized to obtain a dry powder. The dry powder thus obtained was filled in a middle-sized dish made of a resin, and dry press molding was carried out by a known method. Thus, a solid-state solid powder cosmetic was obtained.

As shown in the above Table 5, it was found that in the Production Examples 5-2 to 5-5 in which a fluorine compound was further added in an amount of 0.1% to 10% by mass, and the surfaces of the powder component in the formulation was treated with the fluorine compound, water repellency and oil repellency was improved. On the other hand, when the fluorine compound was added in an amount of 15% by mass (Production Example 5-6), water repellency and oil repellency was excellent, but the product exhibited poor results in terms of easy appliability.

Furthermore, foundations in which a fluorine compound-treated powder was used in combination as the powder component as indicated in the following Formulation Table 6, were prepared, and an evaluation thereof was carried out.

**[Table 6]**

| | Production Example 6-1 | Production Example 6-2 | Production Example 6-3 |
|---|---|---|---|
| Talc | 23 | - | - |
| Sericite | Balance | - | - |
| Mica | 15 | - | - |
| 1H,1H,2H,2H-perfluorooctyltriethoxysilane (5%)-treated talc | - | 23 | - |
| 1H,1H,2H,2H-perfluorooctyltriethoxysilane (5%)-treated sericite | - | Balance | - |
| 1H,1H,2H,2H-perfluorooctyltriethoxysilane (5%)-treated mica | - | 15 | - |
| 1H,1H,2H,2H-perfluorooctyltriethoxysilane (5%)/acrylic-silicone copolymer (formula (V)) (2%)-treated talc | - | - | 23 |
| 1H,1H,2H,2H-perfluorooctyltriethoxysilane (5%)/acrylic-silicone copolymer (formula (V)) (2%)-treated sericite | - | - | Balance |
| 1H,1H,2H,2H-perfluorooctyltriethoxysilane (5%)/acrylic-silicone copolymer (formula (V)) (2%)-treated mica | - | - | 15 |
| Plate-shaped barium sulfate | 5 | 5 | 5 |
| Spherical silicone powder | 10 | 10 | 10 |
| Alkylsilicone-treated iron oxide | 5 | 5 | 5 |
| Titanium oxide fine particles | 5 | 5 | 5 |
| Antiseptic | 0.2 | 0.2 | 0.2 |
| Silicone-treated titanium oxide | 10 | 10 | 10 |
| Amide mixture *1 | 5 | 5 | 5 |
| Diphenylsiloxyphenyltrimethicone | 2 | 2 | 2 |
| Diisostearyl malate | 3 | 3 | 3 |
| Octyl methoxycinnamate | 3 | 3 | 3 |
| Sorbitan sesquiisostearate | 1 | 1 | 1 |
| Water repellency | 3 | 5 | 5 |
| Oil repellency | 3 | 5 | 5 |
| Evaluation of easy appliability | 4 | 4 | 4 |

| | | | |
|---|---|---|---|
| *1: An amide mixture obtained using a mixture of hexamethylenediamine and bisaminomethylcyclohexane, and hydrogenated castor oil fatty acids, by performing an amidation reaction by a conventional method | | | |

(Production method) The various components indicated in the formulations of the above Table 6 were dry mixed and pulverized using a pulverizer, and then the mixture was press molded. Thus, a solid-state solid powder cosmetic was obtained.

As shown in the Table 6, in the Production Examples 6-2 and 6-3 in which a fluorine compound-treated powder obtained by surface-treating with a fluorine compound in advance was incorporated in an amount of about 50% by mass, water repellency and oil repellency was markedly improved. Also, the results for the evaluation of easy appliability were also satisfactory.

Next, foundations prepared by dry mixing using a facing rotor type mixing apparatus as indicated in the following Tables 7 to 9, were evaluated for impact resistance and usage properties (feeling of particulate fineness, moist feeling, and smoothness). The evaluation criteria are as follows. The results are presented together in the following Tables 7 to 9.

### Impact resistance

The foundation of each Test Example was press molded in a resin and was mounted in a compact container for cosmetic use. This was used as a sample. The sample was dropped, while in a state of being laid horizontally, from a height of 30 cm on an iron plate having a thickness of 20 mm, and the number of droppings until breakage was used as an evaluation for impact resistance.

### Usage properties (feeling of particulate fineness, moist feeling, smoothness, mealiness, uniform finish)

A panel of twenty female testers applied the foundation of each Test Example on a half of the face, and performed a comparative evaluation on the feelings of particulate fineness, moist feeling, smoothness, mealiness, and uniform finish.

| | |
|---|---|
| 17 or more people answered good | A |
| 12 to 16 people | B |
| 9 to 11 people | C |
| 5 to 8 people | D |
| 4 or fewer people | E |

**[Table 7]**

| | Production Example 7-1 | Production Example 7-2 |
|---|---|---|
| Talc | Balance | Balance |
| Mica | 10 | 10 |
| Barium sulfate | 5 | 5 |
| Titanium oxide | 20 | 20 |
| Bengala | 0.8 | 0.8 |
| Yellow iron oxide | 2 | 2 |
| Black iron oxide | 0.1 | 0.1 |
| Spherical silicone-based elastomer powder | 5 | 5 |
| Spherical silica | 5 | 5 |
| Spherical nylon powder | 1 | 1 |
| Zinc oxide | 5 | 5 |
| Amide mixture *1 | 5 | 5 |
| Methylphenylpolysiloxane | 3 | 3 |
| Octyl methoxycinnamate | 3 | 3 |
| Petrolatum | 1 | 1 |
| Sorbitan sesquiisostearate | 1 | 1 |
| Paraben | q.s. | q.s. |
| Antioxidant | q.s. | q.s. |
| Production method | Preliminarily mixed in Henschel mixer ↓ Mixed two times with facing rotor type mixing apparatus ↓ Dry press molded in middle-sized resin dish | Preliminarily mixed in Henschel mixer ↓ Mixed two times with pulverizer ↓ Dry press molded in middle-sized resin dish |
| Evaluation of feeling of particulate fineness | A | D |
| Evaluation of moist feeling | B | C |
| Evaluation of smoothness | B | C |
| Evaluation of mealiness | A | D |
| Evaluation of uniform finish | B | C |
| Evaluation of impact resistance | 17 times | 8 times |

| | | |
|---|---|---|
| *1: An amide mixture obtained using a mixture of hexamethylenediamine and bisaminomethylcyclohexane, and hydrogenated castor oil fatty acids, by performing an amidation reaction by a conventional method | | |

### (Production method)

Production Example 7-1: The various components of the formulation were mixed for a certain time in a Henschel mixer (manufactured by Mitsui Miike Machinery Co., Ltd.). Subsequently, the mixture was mixed two times using the facing rotor type mixing apparatus of Fig. 1 (cyclone mill: manufactured by Flo-tec, Ltd.; used by rotating the first rotor and the second rotor in the opposite direction to each other). The mixture was dry press molded in a middle-sized resin dish.

Production Example 7-2: The various components of the formulation were mixed for a certain time in a Henschel mixer. Subsequently, the mixture was mixed two times with a pulverizer (manufactured by Hosokawa Micron Corp.), which is a hammer type pulverizing machine, and was dry press molded in a middle-sized resin dish.

**[Table 8]**

| | Production Example 8-1 | Production Example 8-2 |
|---|---|---|
| Silicone-treated talc | Balance | Balance |
| Silicone-treated mica | 10 | 10 |
| Magnesium myristate-treated barium sulfate | 5 | 5 |
| Aluminum stearate-treated titanium oxide | 20 | 20 |
| Silicone-treated Bengala | 0.8 | 0.8 |
| Silicone-treated tYellow iron oxide | 2 | 2 |
| Silicone-treated black iron oxide | 0.1 | 0.1 |
| Spherical silicone-based elastomer powder | 5 | 5 |
| Spherical silica | 5 | 5 |
| Spherical nylon powder | 1 | 1 |
| Zinc oxide | 5 | 5 |
| Amide mixture * 1 | 5 | 5 |
| Methylphenylpolysiloxane | 3 | 3 |
| Octyl methoxycinnamate | 3 | 3 |
| Petrolatum | 1 | 1 |
| Sorbitan sesquiisostearate | 1 | 1 |
| Paraben | q.s. | q.s. |
| Antioxidant | q.s. | q.s. |
| | Preliminarily mixed in Henschel mixer | Preliminarily mixed in Henschel mixer |
| | ↓ | ↓ |
| Production method | Mixed two times with facing rotor type mixing apparatus | Mixed two times with pulverizer |
| | ↓ | ↓ |
| | Dry press molded in middle-sized resin dish | Dry press molded in middle-sized resin dish |
| Evaluation of feeling of particulate fineness | A | C |
| Evaluation of moist feeling | B | C |
| Evaluation of smoothness | A | B |
| Evaluation of mealiness | A | C |
| Evaluation of uniform finish | B | C |
| Evaluation of impact resistance | 15 times | 7 times |

| | | |
|---|---|---|
| *1: An amide mixture obtained using a mixture of hexamethylenediamine and bisaminomethylcyclohexane, and hydrogenated castor oil fatty acids, by performing an amidation reaction by a conventional method | | |

### (Production method)

Production Example 8-1: The various components of the formulation were mixed for a certain time in a Henschel mixer (manufactured by Mitsui Miike Machinery Co., Ltd.). Subsequently, the mixture was mixed two times using the facing rotor type mixing apparatus of Fig. 1 (cyclone mill: manufactured by Flo-tec, Ltd.; used by rotating the first rotor and the second rotor in the opposite direction to each other). The mixture was dry press molded in a middle-sized resin dish.

Production Example 8-2: The various components of the formulation were mixed for a certain time in a Henschel mixer. Subsequently, the mixture was mixed two times with a pulverizer (manufactured by Hosokawa Micron Corp.), which is a hammer type pulverizing machine, and was dry press molded in a middle-sized resin dish.

**[Table 9]**

| | Production Example 9-1 | Production Example 9-2 |
|---|---|---|
| 1H,1H,2H,2H-perfluorooctyltriethoxysilane (5%)/acrylic-silicone copolymer (formula (V)) (2%)-treated talc | Balance | Balance |
| 1H,1H,2H,2H-perfluorooctyltriethoxysilane (5%)/acrylic-silicone copolymer (formula (V)) (2%)-treated mica | 10 | 10 |
| Barium sulfate | 5 | 5 |
| 1H,1H,2H,2H-perfluorooctyltriethoxysilane (5%)-treated titanium oxide | 20 | 20 |
| 1H,1H,2H,2H-perfluorooctyltriethoxysilane (5%)-treated Bengala | 0.8 | 0.8 |
| 1H,1H,2H,2H-perfluorooctyltriethoxysilane (5%)-treated yellow iron oxide | 2 | 2 |
| 1H,1H,2H,2H-perfluorooctyltriethoxysilane (5%)-treated black iron oxide | 0.1 | 0.1 |
| Spherical silicone-based elastomer powder | 5 | 5 |
| Spherical silica | 5 | 5 |
| Spherical nylon powder | 1 | 1 |
| Zinc oxide | 5 | 5 |
| Amide mixture *1 | 5 | 5 |
| Methylphenylpolysiloxane | 3 | 3 |
| Octyl methoxycinnamate | 3 | 3 |
| Petrolatum | 1 | 1 |
| Sorbitan sesquiisostearate | 1 | 1 |
| Paraben | q.s. | q.s. |
| Antioxidant | q.s. | q.s. |
| | Preliminarily mixed in Henschel mixer | Preliminarily mixed in Henschel mixer |
| Production method | ↓ | ↓ |
| | Mixed two times with facing rotor type mixing apparatus | Mixed two times with pulverizer |
| | ↓ Dry press molded in middle-sized resin dish | ↓ Dry press molded in middle-sized resin dish |
| Evaluation of feeling of particulate fineness | A | D |
| Evaluation of moist feeling | B | C |
| Evaluation of smoothness | B | C |
| Evaluation of mealiness | A | D |
| Evaluation of uniform finish | B | C |
| Evaluation of impact resistance | 17 times | 8 times |

| | | |
|---|---|---|
| *1: An amide mixture obtained using a mixture of hexamethylenediamine and bisaminomethylcyclohexane, and hydrogenated castor oil fatty acids, by performing an amidation reaction by a conventional method | | |

### (Production method)

Production Example 9-1: The various components of the formulation were mixed for a certain time in a Henschel mixer (manufactured by Mitsui Miike Machinery Co., Ltd.). Subsequently, the mixture was mixed two times using the facing rotor type mixing apparatus of Fig. 1 (cyclone mill: manufactured by Flo-tec, Ltd.; used by rotating the first rotor and the second rotor in the opposite direction to each other). The mixture was dry press molded in a middle-sized resin dish.

Production Example 9-2: The various components of the formulation were mixed for a certain time in a Henschel mixer. Subsequently, the mixture was mixed two times with a pulverizer (manufactured by Hosokawa Micron Corp.), which is a hammer type pulverizing machine, and was dry press molded in a middle-sized resin dish.

As shown in the Tables 7 to 9, it was confirmed that the foundations of Production Examples 7-1, 8-1 and 9-1 that were produced using a facing rotor type mixing apparatus had markedly improved impact resistance as compared with the Production Examples 7-2, 8-2 and 9-2 that were produced using a pulverizer (hammer type pulverizing machine). It was also found that the foundations of Production Examples 7-1, 8-1 and 9-1 were also excellent in the usage properties such as the feeling of particulate fineness, moist feeling, smoothness, mealiness, and uniform finish.

The same evaluations were performed on the foundations produced by using a facing rotor type mixing apparatus and varying the amount of incorporation of the amide mixture between 0.1% and 13% by mass. The results are presented in the following Table 10.

**[Table 10]**

| | Production Example 10-1 | Production Example 10-2 | Production Example 10-3 | Production Example 10-4 | Production Example 10-5 | Production Example 10-6 | Production Example 10-7 |
|---|---|---|---|---|---|---|---|
| Talc | Balance | Balance | Balance | Balance | Balance | Balance | Balance |
| Mica | 10 | 10 | 10 | 10 | 10 | 10 | 10 |
| Barium sulfate | 5 | 5 | 5 | 5 | 5 | 5 | 5 |
| Titanium oxide | 20 | 20 | 20 | 20 | 20 | 20 | 20 |
| Bengala | 0.8 | 0.8 | 0.8 | 0.8 | 0.8 | 0.8 | 0.8 |
| Yellow iron oxide | 2 | 2 | 2 | 2 | 2 | 2 | 2 |
| Black iron oxide | 0.1 | 0.1 | 0.1 | 0.1 | 0.1 | 0.1 | 0.1 |
| Spherical silicone-based elastomer powder | 5 | 5 | 5 | 5 | 5 | 5 | 5 |
| Spherical silica | 5 | 5 | 5 | 5 | 5 | 5 | 5 |
| Spherical nylon powder | 1 | 1 | 1 | 1 | 1 | 1 | 1 |
| Zinc oxide | 5 | 5 | 5 | 5 | 5 | 5 | 5 |
| Amide mixture *1 | 0.1 | 1 | 3 | 5 | 7 | 10 | 13 |
| Methylphenylpolysiloxane | 3 | 3 | 3 | 3 | 3 | 3 | 3 |
| Octyl methoxycinnamate | 3 | 3 | 3 | 3 | 3 | 3 | 3 |
| Petrolatum | 1 | 1 | 1 | 1 | 1 | 1 | 1 |
| Sorbitan sesquiisostearate | 1 | 1 | 1 | 1 | 1 | 1 | 1 |
| Paraben | q.s. | q.s. | q.s. | q.s. | q.s. | q.s. | q.s. |
| Antioxidant | q.s. | q.s. | q.s. | q.s. | q.s. | q.s. | q.s. |
| | Preliminarily mixed in Henschel mixer | | | | | | |
| | ↓ | | | | | | |
| Production method | Mixed two times with facing rotor type mixing apparatus | | | | | | |
| | ↓ | | | | | | |
| | Dry press molded in middle-sized resin dish | | | | | | |
| Evaluation of feeling of particulate fineness | A | A | A | A | A | B | D |
| Evaluation of moist feeling | C | C | B | B | B | A | A |
| Evaluation of smoothness | A | B | B | B | B | C | E |
| Evaluation of mealiness | D | C | B | A | B | B | B |
| Evaluation of uniform finish | D | B | B | B | B | C | E |
| Evaluation of impact resistance | 8 times | 10 times | 13 times | 17 times | 20 times | 22 times | 25 times |

| | | | | | | | |
|---|---|---|---|---|---|---|---|
| *1: An amide mixture obtained using a mixture of hexamethylenediamine and bisaminomethylcyclohexane, and hydrogenated castor oil fatty acids, by performing an amidation reaction by a conventional method | | | | | | | |

(Production method) The various components of each formulation in the Table 10 were mixed for a certain time in a Henschel mixer (manufactured by Mitsui Miike Machinery Co., Ltd.). Subsequently, the mixture was mixed two times using the facing rotor type mixing apparatus of Fig. 1 (cyclone mill: manufactured by Flo-tec, Ltd.; used by rotating the first rotor and the second rotor in the opposite direction to each other). The mixture was dry press molded in a middle-sized resin dish.

As shown in the Table 10, the foundations of Production Examples 10-2 to 10-7 in which the amide mixture was incorporated in an amount of 1% to 10% by mass, had excellent impact resistance and were also satisfactory in terms of usage properties such as the feeling of particulate fineness, moist feeling, smoothness, mealiness, and uniform finish. On the contrary, in the Production Example 10-1 in which the amide mixture was not incorporated, a product that was particularly satisfactory in terms of mealiness and uniform finish, could not be obtained, and impact resistance could not be said to be sufficiently improved. The Production Example 10-7 in which the amide mixture was incorporated in an amount of 13% by mass, was particularly inferior in terms of smoothness and uniform finish.

Based on these results, particularly in the case of producing the solid powder cosmetic of the present invention using a facing rotor type mixing apparatus, the amount of incorporation of the amide mixture is suitably 1% to 10% by mass.

Hereinafter, other formulation examples of the solid powder cosmetic according to the present invention will be described below. The amounts in the following formulations are expressed in percent (%) by mass.

### Powdery foundation I

The various components shown in the following formulation were mixed for a certain time with a Henschel mixer, and then the components were mixed two times using the facing rotor type mixing apparatus of Fig. 1. The mixture was dry press molded in a middle-sized resin dish, and thus a powdery foundation was obtained. The powdery foundation thus obtained was excellent in all of the long-lasting makeup effect, usage properties, and impact resistance.

| Formulation | |
|---|---|
| (Powder component) | |
| 1H,1H,2H,2H-perfluorooctyltriethoxysilane (5%)-treated talc | Balance |
| 1H,1H,2H,2H-perfluorooctyltriethoxysilane (5%)-treated synthetic mica | 10 |
| Plate-shaped barium sulfate | 5 |
| Alkyl-treated titanium oxide | 10 |
| Fatty acid aluminum-treated titanium oxide fine particles | 5 |
| 1H,1H,2H,2H-perfluorooctyltriethoxysilane (5%)-treated black titanium oxide fine particles | 2 |
| Alkyl-treated iron oxide | 4 |
| Silicone powder | 10 |
| Spherical silica | 5 |
| Spherical nylon | 1 |
| Activated zinc oxide | 5 |
| Spherical porous PMMA resin powder *1 | 5 |
| (Oily component) | |
| Methylphenylsilicone oil | 5 |
| Ester oil | 3 |
| Amide mixture *2 | 4 |
| (Others) | |
| Antiseptic | 0.2 |
| Ultraviolet absorber | 4 |
| Antioxidant | 0.1 |
| Active agent | 1 |

| | |
|---|---|
| *1: TECHPOLYMER MBP-8HP; manufactured by Sekisui Chemical Co., Ltd. (average particle size 8 µm, specific surface area 150 m²/g, most frequent pore diameter 180 Å) *2: An amide mixture obtained using a mixture of hexamethylenediamine and bisaminomethylcyclohexane, and hydrogenated castor oil fatty acids, by performing an amidation reaction by a conventional method | |

### Powdery foundation II

The various components shown in the following formulation (except for the pearl pigment component) were mixed for a certain time with a Henschel mixer, and then the components were mixed two times using the facing rotor type mixing apparatus of Fig. 1. The pearl pigment was added thereto, and the mixture was lightly pulverized. Subsequently, the pulverization product was dry press molded in a middle-sized resin dish to obtain a powdery foundation. The powdery foundation thus obtained was excellent in the long-lasting makeup effect, usage properties, and impact resistance, as well as in the pearl-like texture.

| •Formulation | |
|---|---|
| (Powder component) | |
| Talc | Balance |
| Synthetic fluorophlogopite | 20 |
| Sericite | 30 |
| Boron nitride | 4 |
| Zinc myristate | 3 |
| Silicone-treated titanium oxide | 15 |
| Silicone-treated red iron oxide | 1 |
| Silicone-treated yellow iron oxide | 3 |
| Silicone-treated black iron oxide | 0.3 |
| (Pearl pigment component) | |
| Spherical barium sulfate-coated red interference titanated mica | 3 |
| Spherical barium sulfate-coated yellow interference titanated mica | |
| | 2 |
| (Oily component) | |
| Triisostearin | 2 |
| Petrolatum | 2 |
| Trioctanoin | 2 |
| Dimethicone | 3 |
| Sorbitan sesquiisostearate | 0.8 |
| Amide mixture *1 | 4 |
| (Others) | |
| Antiseptic | q.s. |
| Antioxidant | q.s. |
| Fragrance | q.s. |

| | |
|---|---|
| *1: An amide mixture obtained using a mixture of hexamethylenediamine and bisaminomethylcyclohexane, and hydrogenated castor oil fatty acids, by performing an amidation reaction by a conventional method | |

### Powdery foundation III

The various components shown in the following formulation were dispersed and mixed in ethyl alcohol with a disper-mixer, and the slurry viscosity was adjusted to about 2000 mPa·s. Subsequently, the slurry was subjected to cracking/pulverization/dispersion using a sand grinder mill filled with 2-mmφ zirconia beads. The powder slurry thus obtained was dried with a spin flash dryer to volatilize ethyl alcohol, and thus a dry powder was obtained. The dry powder thus obtained was filled in a middle-sized dish container made of a resin, and was press molded by a conventional dry press method. Thus, a powdery foundation was obtained. The powdery foundation thus obtained was excellent in both the long-lasting makeup effect and usage properties.

| •Formulation | |
|---|---|
| (Powder component) | |
| Silicone-treated talc | Balance |
| Calcined mica | 15 |
| Synthetic mica | 10 |
| Plate-shaped barium sulfate | 5 |
| Silicone-treated titanium oxide | 10 |
| Silicone-treated fusiform titanium oxide | 15 |
| Hydrophobized iron oxide | 4 |
| Spherical silicone powder | 3 |
| Spherical PMMA powder | 5 |
| Spherical silica | 1 |
| Activated zinc oxide | 5 |
| (Oily component) | |
| Cyclomethicone | 4 |
| Diphenylsiloxyphenyltrimethicone | 2 |
| Petrolatum | 2 |
| Octyl methoxycinnamate | 7 |
| Octocrylene | 5 |
| Silicone-based film-forming agent | 4 |
| Amide mixture *1 | 1 |
| 1H,1H,2H,2H-perfluorooctyltriethoxysilane | 1 |
| (Others) | |
| Antiseptic | 0.1 |
| Active agent | 1 |

| | |
|---|---|
| *1: An amide mixture obtained using a mixture of hexamethylenediamine and bisaminomethylcyclohexane, and hydrogenated castor oil fatty acids, by performing an amidation reaction by a conventional method | |

### Powdery foundation II

The powder component (except for the pearl pigment component), the oily component, and the fluorine compound of the present invention shown in the following formulation were mixed, and the mixture was mixed in isopropyl alcohol with a disper-mixer. The slurry viscosity was adjusted to about 2000 mPa·s, and then the slurry was subjected to cracking/pulverization/dispersion using a sand grinder mill filled with 2-mmφ zirconia beads. The powder slurry thus obtained was dried with a spin flash dryer, and a dry powder thus obtained and the pearl pigment portion were mixed with a Henschel mixer. A powder thus obtained was filled in a container made of a resin, and thus a powdery foundation was obtained. The powdery foundation thus obtained was excellent in the feeling of use and the pearl-like texture.

### Dual foundation

The various components shown in the following formulation were mixed with a Henschel mixer, and then the mixture was mixed two times using the facing rotor type mixing apparatus of Fig. 1. The resulting mixture was dry press molded in a middle-sized resin dish, and thus, a dual foundation was obtained. The dual foundation thus obtained was excellent in all of the long-lasting makeup effect, usage properties, and impact resistance.

| •Formulation | |
|---|---|
| (Powder component) | |
| Silicone-treated talc | Balance |
| Silicone-treated sericite | 20 |
| Silicone-treated mica | 10 |
| Silicone-treated titanium oxide | 10 |
| Zinc oxide | 5 |
| Plate-shaped silicic anhydride | 5 |
| Silicone-treated Bengala | 0.8 |
| Silicone-treated yellow iron oxide | 3 |
| Silicone-treated black iron oxide | 0.2 |
| Spherical silicone elastomer powder | 5 |
| Spherical silicone resin-coated silicone elastomer powder 5 | |
| (Oily component) | |
| Liquid paraffin | 4 |
| Petrolatum | 4 |
| Sorbitan sesquiisostearate | 0.8 |
| Amide mixture *1 | 4 |
| (Others) | |
| Antiseptic | q.s. |
| Antioxidant | q.s. |
| Fragrance | q.s |

| | |
|---|---|
| *1: An amide mixture obtained using a mixture of hexamethylenediamine and bisaminomethylcyclohexane, and hydrogenated castor oil fatty acids, by performing an amidation reaction by a conventional method | |

### Loose powder

The various components shown in the following formulation (except for the pearl pigment component) were mixed for a certain time with a Henschel mixer, and then the mixture was mixed two times using the facing rotor type mixing apparatus of Fig. 1. The pearl pigment was added thereto, and the mixture was lightly pulverized. Subsequently, the resulting mixture was dry press molded in a middle-sized resin dish, and thus, a loose powder was obtained. The loose powder thus obtained was excellent in all of the long-lasting makeup effect, usage properties, and impact resistance, as well as in the pearl-like texture.

| •Formulation | |
|---|---|
| (Powder component) | |
| Mica | 10 |
| Talc | Balance |
| Zinc oxide | 5 |
| Titanium oxide fine particles | 3 |
| Spherical silicone powder | 20 |
| (Pearl pigment) | |
| Iron oxide-coated red interference titanated mica | 10 |
| (Oily component) | |
| Petrolatum | 1 |
| Squalane | 2 |
| Diisostearyl malate | 1 |
| Amide mixture *1 | 3 |
| (Others) | |
| Antiseptic | q.s. |
| Antioxidant | q.s. |
| Fragrance | q.s. |

| | |
|---|---|
| *1: An amide mixture obtained using a mixture of hexamethylenediamine and bisaminomethylcyclohexane, and hydrogenated castor oil fatty acids, by performing an amidation reaction by a conventional method | |

### Eye shadow

The various components shown in the following formulation (except for the pearl pigment component) were mixed for a certain time with a Henschel mixer, and then the mixture was mixed two times using the facing rotor type mixing apparatus of Fig. 1. The pearl pigment was added thereto, and the mixture was lightly pulverized. Subsequently, the resulting mixture was dry press molded in a middle-sized resin dish, and thus, an eye shadow was obtained. An eye shadow was obtained. The eye shadow thus obtained was excellent in all of the long-lasting makeup effect, usage properties, and impact resistance, as well as in the pearl-like texture.

| •Formulation | |
|---|---|
| (Powder component) | |
| Talc | Balance |
| Calcined sericite | 30 |
| (Pearl pigment portion) | |
| Iron oxide-coated titanated mica | 30 |
| Silicic anhydride-coated aluminum Bengala | 10 |
| (Oily component) | |
| Petrolatum | 5 |
| Diisostearyl malate | 5 |
| Sorbitan sesquiisostearate | 0.8 |
| Amide mixture *1 | 1 |
| (Others) | |
| Methylparaben | q.s. |
| Antioxidant | q.s. |
| Fragrance | q.s. |

| | |
|---|---|
| *1: An amide mixture obtained using a mixture of hexamethylenediamine and bisaminomethylcyclohexane, and hydrogenated castor oil fatty acids, by performing an amidation reaction by a conventional method | |

### Pressed powder

The various components shown in the following formulation were mixed with a Henschel mixer, and then the mixture was mixed two times using the facing rotor type mixing apparatus of Fig. 1. The resulting mixture was dry press molded in a middle-sized resin dish, and thus, a pressed powder was obtained. The pressed powder thus obtained was excellent in all of the long-lasting makeup effect, usage properties, and impact resistance.

| •Formulation | |
|---|---|
| (Powder component) | |
| Metal soap-treated talc | Balance |
| Synthetic fluorophlogopite | 10 |
| Spherical urethane powder | 5 |
| Spherical porous PMMA resin powder *1 | 5 |
| L-lauroyllysine | 5 |
| (Oily component) | |
| Squalane | 2 |
| Polybutene | 1 |
| Dimethicone | 2 |
| Sorbitan sesquiisostearate | 0.8 |
| Amide mixture *2 | 1 |
| (Others) | |
| Antiseptic | q.s. |
| Antioxidant | q.s. |
| Fragrance | q.s. |

| | |
|---|---|
| *1: TECHPOLYMER MBP-8HP; manufactured by Sekisui Chemical Co., Ltd. (average particle size 8 µm, specific surface area 150 m²/g, most frequent pore diameter 180 Å) *2: An amide mixture obtained using a mixture of hexamethylenediamine and bisaminomethylcyclohexane, and hydrogenated castor oil fatty acids, by performing an amidation reaction by a conventional method | |

### Body powder

The various components shown in the following formulation were mixed with a Henschel mixer, and then the mixture was mixed two times using the facing rotor type mixing apparatus of Fig. 1. The resulting mixture was dry press molded in a middle-sized resin dish, and thus, a body powder was obtained. The body powder thus obtained had excellent usage properties.

| •Formulation | |
|---|---|
| (Powder component) | |
| Talc | Balance |
| Mica | 10 |
| Zinc oxide | 5 |
| Spherical silicone powder | 20 |
| (Oily component) | |
| Petrolatum | 1 |
| Squalane | 2 |
| Diisostearyl malate | 1 |
| Amide mixture *1 | 1 |
| (Others) | |
| Antiseptic | q.s. |
| Antioxidant | q.s. |
| Fragrance | q.s. |

| | |
|---|---|
| *1: An amide mixture obtained using a mixture of hexamethylenediamine and bisaminomethylcyclohexane, and hydrogenated castor oil fatty acids, by performing an amidation reaction by a conventional method | |

### Perfume powder

The various components shown in the following formulation were mixed with a Henschel mixer, and then the mixture was mixed two times using the facing rotor type mixing apparatus of Fig. 1. The resulting mixture was dry press molded in a middle-sized resin dish, and thus, a perfume powder was obtained. The perfume powder thus obtained had excellent usage properties.

| •Formulation | |
|---|---|
| (Powder component) | |
| Silicone-treated talc | Balance |
| Synthetic fluorophlogopite | 10 |
| Boron nitride | 2 |
| Spherical silicone elastomer powder | 20 |
| Red No. 226 | 0.1 |
| (Oily component) | |
| Petrolatum | 2 |
| Trioctanoin | 2 |
| Amide mixture *1 | 1 |
| (Others) | |
| Antiseptic | q.s. |
| Antioxidant | q.s. |
| Fragrance | 1 |

| | |
|---|---|
| *1: An amide mixture obtained using a mixture of hexamethylenediamine and bisaminomethylcyclohexane, and hydrogenated castor oil fatty acids, by performing an amidation reaction by a conventional method | |

### DESCRIPTION OF REFERENCE NUMBERS

- 10: FACING ROTOR TYPE MIXING APPARATUS
- 11: MIXING CHAMBER
- 12: MOTOR
- 13: MOTOR
- 14: FIRST ROTOR
- 15: SECOND ROTOR
- 16: FEED PORT
- 17: DISCHARGE PORT
- 20: RAW MATERIAL SUPPLYING DEVICE
- 30: CAPTURING DEVICE
- 32: COLLECTING CONTAINER
- 40: SUCTION DEVICE
- 112: STORAGE TANK
- 114: DRYING APPARATUS
- 116: HOUSING
- 118: SHEARING UNIT
- 120: SUPPLY UNIT
- 122: GAS BLOWING UNIT
- 124: CAPTURING UNIT
- 210: MEDIUM AGITATING MILL

## Claims

1. A solid powder cosmetic comprising:
a powder component,
an oily component as a binder, and
an amide mixture obtained by amidating a mixture of hexamethylenediamine and
bisaminomethylcyclohexane with hydrogenated castor oil fatty acids.

2. The solid powder cosmetic according to claim 1, wherein an amount of the amide mixture is 1.0% to 15% by mass.

3. The solid powder cosmetic according to claim1 or 2, comprising 1% to 20% by mass of spherical poly(meth)acrylate particles as the powder component.

4. The solid powder cosmetic according to claim3, wherein the spherical poly(meth)acrylate particles are particles having an average particle size of 3 to 20 µm, a specific surface area of 80 to 180 m²/g, and a most frequent pore diameter of 180 Å or larger.

5. The solid powder cosmetic according to any of claim1 to 4, further comprising an organically modified clay mineral in an amount of 1.0% to 15% by mass as a total amount with the amide mixture.

6. The solid powder cosmetic according to any of claim1 to 5, comprising 5% to 97% by mass of fluorine compound-treated powder as the powder component.

7. The solid powder cosmetic according to claim6, wherein the fluorine compound is 1H,1H,2H,2H-perfluorooctyltriethoxysilane.

8. A method for producing a solid powder cosmetic comprising:
mixing step of mixing a powder component, an oily component as a binder, and an amide mixture obtained by amidating a mixture of hexamethylenediamine and bisaminomethylcyclohexane with hydrogenated castor oil fatty acids, and
producing a solid powder cosmetic from the obtained powder mixture,
wherein the mixing step is carried out by using a facing rotor type mixing apparatus which has a first rotor provided with plural blades and a second rotor provided with plural blades, disposed in a mixing chamber such that the first rotor and the second rotor face each other and respectively have an individual rotating shaft on the same axis line in an approximately horizontal direction, and which mixes raw materials by rotating the first rotor and the second rotor in the same or opposite direction to each other, while supplying the raw materials through a feed port on the first rotor side, and discharges the mixed raw materials through a discharge port on the second rotor side.

9. The method for producing a solid powder cosmetic according to claim8, wherein the first rotor and the second rotor of the facing rotor type mixing apparatus rotate in the opposite direction to each other.

10. The method for producing a solid powder cosmetic according to claim 8 or 9, wherein an amount of the amide mixture is 1.0% to 15% by mass.

11. The method for producing a solid powder cosmetic according to any of claim 8 to 10, mixing with 1% to 20% by mass of spherical poly(meth)acrylate particles as the powder component.

12. The method for producing a solid powder cosmetic according to claim 11, wherein the spherical poly(meth)acrylate particles are the particles having an average particle size of 3 to 20 µm, a specific surface area of 80 to 180 m²/g, and a most frequent pore diameter of 180 Å or larger.

13. The method for producing a solid powder cosmetic according to any of claim 8 to 12, further mixing with an organically modified clay mineral in an amount of 1.0% to 15% by mass as a total amount with the amide mixture.

14. The method for producing a solid powder cosmetic according to any of claim 8 to 13, mixing with 5% to 97% by mass of fluorine compound-treated powder as the powder component.

15. The method for producing a solid powder cosmetic according to claim 14, wherein the fluorine compound is 1H,1H,2H,2H-perfluorooctyltriethoxysilane.

16. A method for producing a solid powder cosmetic comprising:
a slurry preparation step of mixing a powder component, an oily component as a binder, and
an amide mixture obtained by amidating a mixture of hexamethylenediamine and bisaminomethylcyclohexane with hydrogenated castor oil fatty acids, in a volatile solvent to obtain a slurry; and
a drying step of drying the slurry to obtain a dry powder, and
producing a solid powder cosmetic from the obtained dry powder,
wherein the drying step is carried out by using a drying apparatus which performs drying of the slurry by converting the slurry into fine liquid droplets by means of a mechanical shear force and blowing a dry gas to the fine liquid droplets.

17. The method for producing a solid powder cosmetic according to claim 16, wherein the drying apparatus is an apparatus having a hollow-shaped housing; a shearing unit that shears a slurry by means of shearing members provided inside the housing and converts the slurry into fine liquid droplets; a supply unit that supplies the slurry to the shearing members in the housing; a gas blowing unit that blows a dry gas into the housing, and supplying the dry gas to the slurry that has been converted to fine liquid droplets by the shearing unit to bring the dry gas and the fine liquid droplets into contact; and a capture unit that captures a dry powder produced by drying the slurry.

18. The method for producing a solid powder cosmetic according to claim16 or 17, in the slurry preparation step, obtaining a slurry by mixing the powder component and the oily component in a volatile solvent using a medium agitating mill, and cracking and/or pulverizing and/or dispersing the powder component.

19. The method for producing a solid powder cosmetic according to any of claim 16 to 18, wherein an amount of the amide mixture is 1.0% to 15% by mass.

20. The method for producing a solid powder cosmetic according to any of claim 16 to 19, mixing with 1% to 20% by mass of spherical poly(meth)acrylate particles as the powder component.

21. The method for producing a solid powder cosmetic according to claim 20, wherein the spherical poly(meth)acrylate particles are the particles having an average particle size of 3 to 20 µm, a specific surface area of 80 to 180 m²/g, and a most frequent pore diameter of 180 Å or larger.

22. The method for producing a solid powder cosmetic according to any of claim 16 to 21, further mixing with an organically modified clay mineral in an amount of 1.0% to 15% by mass as a total amount with the amide mixture.

23. The method for producing a solid powder cosmetic according to any of claim 16 to 22, mixing with 5% to 97% by mass of fluorine compound-treated powder as the powder component.

24. The method for producing a solid powder cosmetic according to any of claim 16 to 22, in the slurry preparation step, mixing with 0.1 % to 10% by mass of fluorine compound.

25. The method for producing a solid powder cosmetic according to claim23 or 24, wherein the fluorine compound is 1H,1H,2H,2H-perfluorooctyltriethoxysilane.

## Patentansprüche

1. Feststoffpulver-Kosmetikum, umfassend:
eine Pulverkomponente,
eine Ölkomponente als Bindemittel, und
ein Amidgemisch, welches durch Amidieren eines Gemisches aus Hexamethylendiamin und Bis(aminomethylcyclohexan) mit hydrierten Castorölfettsäuren erhalten worden ist.

2. Feststoffpulver-Kosmetikum nach Anspruch 1, wobei die Menge an Amidgemisch 1.0 Masse% bis 15 Masse% beträgt.

3. Feststoffpulver-Kosmetikum nach Anspruch 1 oder 2, umfassend 1 Masse% bis 20 Masse% an kugelförmigen Poly(meth)acrylat-Partikeln als Pulverkomponente.

4. Feststoffpulver-Kosmetikum nach Anspruch 3, wobei es sich bei den kugelförmigen Poly(meth)acrylat-Partikeln um Partikel handelt, welche eine mittlere Partikelgröße von 3 bis 20 µm, eine spezifische Oberfläche von 80 bis 180 m²/g, sowie einen häufigsten Porendurchmesser von 180 Å oder mehr aufweisen.

5. Feststoffpulver-Kosmetikum nach einem der Ansprüche 1 bis 4, weiterhin umfassend ein organisch-modifiziertes Tonmineral in einer Menge von 1.0 Masse% bis 15 Masse%, angegeben als Gesamtmenge zusammen mit dem Amidgemisch.

6. Feststoffpulver-Kosmetikum nach einem der Ansprüche 1 bis 5, umfassend 5 Masse% bis 97 Masse% eines mit einer Fluorverbindung behandelten Pulvers als Pulverkomponente.

7. Feststoffpulver-Kosmetikum nach Anspruch 6, wobei es sich bei der Fluorverbindung um 1H,1H,2H,2H-Perfluoroctyltriethoxysilan handelt.

8. Verfahren zur Herstellung eines Feststoffpulver-Kosmetikums, umfassend:
einen Mischschritt, welcher das Mischen einer Pulverkomponente, einer als Bindemittel fungierenden Ölkomponente, sowie eines durch Amidieren eines Gemisches aus Hexamethylendiamin und Bis(aminomethylcyclohexan) mit hydrierten Castorölfettsäuren erhaltenen Amidgemisches vorsieht, und
Herstellen eines Feststoffpulver-Kosmetikums aus dem erhaltenen Pulvergemisch,
wobei der Mischschritt unter Verwendung einer mit gegenüberliegenden Rotoren ausgestatteten Mischvorrichtung durchgeführt wird, welche einen mit mehreren Blättern ausgestatteten ersten Rotor und einen mit mehreren Blättern ausgestatteten zweiten Rotor aufweist, wobei diese derart in einer Mischkammer angeordnet sind, dass der erste Rotor und der zweite Rotor einander gegenüberliegen und auf der gleichen Achsenlinie in annähernd horizontaler Richtung jeweils eine einzelne Drehwelle aufweisen, und welche die Rohmaterialien durch Drehen des ersten Rotors und des zweiten Rotors in die gleiche Richtung oder in entgegengesetzte Richtungen miteinander vermischt, während die Rohmaterialien über eine auf der Seite des ersten Rotors befindliche Einlassöffnung zugeführt und die miteinander vermischten Rohmaterialien über eine auf der Seite des zweiten Rotors befindliche Auslassöffnung abgeführt werden.

9. Verfahren zur Herstellung eines Feststoffpulver-Kosmetikums nach Anspruch 8, wobei sich der erste Rotor und der zweite Rotor der mit gegenüberliegenden Rotoren ausgestatteten Mischvorrichtung in entgegengesetzte Richtungen drehen.

10. Verfahren zur Herstellung eines Feststoffpulver-Kosmetikums nach Anspruch 8 oder 9, wobei die Menge an Amidgemisch 1.0 Masse% bis 15 Masse% beträgt.

11. Verfahren zur Herstellung eines Feststoffpulver-Kosmetikums nach einem der Ansprüche 8 bis 10, welches das Einmischen von 1 Masse% bis 20 Masse% an kugelförmigen Poly(meth)acrylat-Partikeln als Pulverkomponente vorsieht.

12. Verfahren zur Herstellung eines Feststoffpulver-Kosmetikums nach Anspruch 11, wobei es sich bei den kugelförmigen Poly(meth)acrylat-Partikeln um Partikel handelt, welche eine mittlere Partikelgröße von 3 bis 20 µm, eine spezifische Oberfläche von 80 bis 180 m²/g, sowie einen häufigsten Porendurchmesser von 180 Å oder mehr aufweisen.

13. Verfahren zur Herstellung eines Feststoffpulver-Kosmetikums nach einem der Ansprüche 8 bis 12, welches weiterhin das Einmischen eines organisch-modifizierten Tonminerals in einer Menge von 1.0 Masse% bis 15 Masse%, angegeben als Gesamtmenge zusammen mit dem Amidgemisch, vorsieht.

14. Verfahren zur Herstellung eines Feststoffpulver-Kosmetikums nach einem der Ansprüche 8 bis 13, welches das Einmischen von 5 Masse% bis 97 Masse% eines mit einer Fluorverbindung behandelten Pulvers als Pulverkomponente vorsieht.

15. Verfahren zur Herstellung eines Feststoffpulver-Kosmetikums nach Anspruch 14, wobei es sich bei der Fluorverbindung um 1H,1H,2H,2H-Perfluoroctyltriethoxysilan handelt.

16. Verfahren zur Herstellung eines Feststoffpulver-Kosmetikums, umfassend:
einen Aufschlämmungsherstellungsschritt, welcher das Mischen einer Pulverkomponente, einer als Bindemittel fungierenden Ölkomponente, sowie eines durch Amidieren eines Gemisches aus Hexamethylendiamin und Bis(aminomethylcyclohexan) mit hydrierten Castorölfettsäuren erhaltenen Amidgemisches in einem flüchtigen Lösungsmittel unter Bereitstellung einer Aufschlämmung vorsieht, und
einen Trocknungsschritt, welcher das Trocknen der Aufschlämmung unter Bereitstellung eines Trockenpulvers vorsieht, und
Herstellen eines Feststoffpulver-Kosmetikums aus dem erhaltenen Trockenpulver,
wobei der Trocknungsschritt unter Verwendung einer Trocknungsvorrichtung durchgeführt wird, in welcher die Trocknung der Aufschlämmung dadurch erfolgt, dass die Aufschlämmung mittels mechanischer Scherkräfte in feine Tröpfchen umgewandelt wird und die feinen Tröpfchen mit einem trockenen Gas angeblasen werden.

17. Verfahren zur Herstellung eines Feststoffpulver-Kosmetikums nach Anspruch 16, wobei es sich bei der Trocknungsvorrichtung um eine Vorrichtung handelt, welche ein hohlförmiges Gehäuse; eine Schereinheit, mittels welcher eine Aufschlämmung unter Verwendung von im Inneren des Gehäuses bereitgestellten Scherelementen einer Scherung ausgesetzt wird und welche die Aufschlämmung in feine Tröpfchen umwandelt; eine Zuführeinheit, mittels welcher die Aufschlämmung den im Gehäuse befindlichen Scherelementen zugeführt wird; eine Gaseinblaseinheit, mittels welcher ein trockenes Gas in das Gehäuse eingeblasen und das trockene Gas der Aufschlämmung, die unter Verwendung der Schereinheit in feine Tröpfchen umgewandelt worden ist, zugeführt wird, um das trockene Gas und die feinen Tröpfchen miteinander in Kontakt zu bringen; sowie eine Auffangeinheit, mittels welcher ein durch Trocknen der Aufschlämmung erzeugtes Trockenpulver aufgefangen wird, aufweist.

18. Verfahren zur Herstellung eines Feststoffpulver-Kosmetikums nach Anspruch 16 oder 17, welches im Rahmen des Aufschlämmungsherstellungsschrittes das Bereitstellen einer Aufschlämmung durch Mischen der Pulverkomponente und der Ölkomponente in einem flüchtigen Lösungsmittel unter Verwendung einer das Medium rührenden Mühle, sowie das Zerbrechen und/oder Pulverisieren und/oder Dispergieren der Pulverkomponente vorsieht.

19. Verfahren zur Herstellung eines Feststoffpulver-Kosmetikums nach einem der Ansprüche 16 bis 18, wobei die Menge an Amidgemisch 1.0 Masse% bis 15 Masse% beträgt.

20. Verfahren zur Herstellung eines Feststoffpulver-Kosmetikums nach einem der Ansprüche 16 bis 19, welches das Einmischen von 1 Masse% bis 20 Masse% an kugelförmigen Poly(meth)acrylat-Partikeln als Pulverkomponente vorsieht.

21. Verfahren zur Herstellung eines Feststoffpulver-Kosmetikums nach Anspruch 20, wobei es sich bei den kugelförmigen Poly(meth)acrylat-Partikeln um Partikel handelt, welche eine mittlere Partikelgröße von 3 bis 20 µm, eine spezifische Oberfläche von 80 bis 180 m²/g, sowie einen häufigsten Porendurchmesser von 180 Å oder mehr aufweisen.

22. Verfahren zur Herstellung eines Feststoffpulver-Kosmetikums nach einem der Ansprüche 16 bis 21, welches weiterhin das Einmischen eines organisch-modifizierten Tonminerals in einer Menge.von 1.0 Masse% bis 15 Masse%, angegeben als Gesamtmenge zusammen mit dem Amidgemisch, vorsieht.

23. Verfahren zur Herstellung eines Feststoffpulver-Kosmetikums nach einem der Ansprüche 16 bis 22, welches das Einmischen von 5 Masse% bis 97 Masse% eines mit einer Fluorverbindung behandelten Pulvers als Pulverkomponente vorsieht.

24. Verfahren zur Herstellung eines Feststoffpulver-Kosmetikums nach einem der Ansprüche 16 bis 22, welches im Rahmen des Aufschlämmungsherstellungsschrittes das Einmischen von 0.1 Masse% bis 10 Masse% an Fluorverbindung vorsieht.

25. Verfahren zur Herstellung eines Feststoffpulver-Kosmetikums nach Anspruch 23 oder 24, wobei es sich bei der Fluorverbindung um 1H,1H,2H,2H-Perfluoroctyltriethoxysilan handelt.

## Revendications

1. Produit cosmétique sous forme de poudre solide, comprenant :
un composant sous forme de poudre,
un composant huileux en tant que liant, et
un mélange d'amide obtenu en amidant un mélange d'hexaméthylènediamine et de bisaminométhylcyclohexane avec des acides gras d'huile de ricin hydrogénée.

2. Produit cosmétique sous forme de poudre solide selon la revendication 1, dans lequel une quantité du mélange d'amide est de 1,0 % à 15 % en masse.

3. Produit cosmétique sous forme de poudre solide selon la revendication 1 ou 2, comprenant de 1 % à 20 % en masse de particules sphériques de poly(méth)acrylate en tant que composant sous forme de poudre.

4. Produit cosmétique sous forme de poudre solide selon la revendication 3, dans lequel les particules sphériques de poly(méth)acrylate sont des particules ayant une taille moyenne de particule de 3 à 20 µm, une surface spécifique de 80 à 180 m²/g et un diamètre de pore le plus fréquent de 180 Å ou plus.

5. Produit cosmétique sous forme de poudre solide selon l'une quelconque des revendications 1 à 4, comprenant en outre un minéral argileux modifié organiquement en une quantité de 1,0 % à 15 % en masse en tant que quantité totale avec le mélange d'amide.

6. Produit cosmétique sous forme de poudre solide selon l'une quelconque des revendications 1 à 5, comprenant de 5 % à 97 % en masse de poudre traitée par un composé fluoré en tant que composant sous forme de poudre.

7. Produit cosmétique sous forme de poudre solide selon la revendication 6, dans lequel le composé fluoré est le 1H,1H,2H,2H-perfluorooctyltriéthoxysilane.

8. Procédé pour fabriquer un produit cosmétique sous forme de poudre solide, comprenant :
une étape de mélange consistant à mélanger un composant sous forme de poudre, un composant huileux en tant que liant, et un mélange d'amide obtenu en amidant un mélange d'hexaméthylènediamine et de bisaminométhylcyclohexane avec des acides gras d'huile de ricin hydrogénée, et
la production d'un produit cosmétique sous forme de poudre solide à partir du mélange sous forme de poudre obtenu,
dans lequel l'étape de mélange est mise en oeuvre en utilisant un appareil de mélange de type à rotors opposés qui a un premier rotor doté de plusieurs lames et un second rotor doté de plusieurs lames, disposé dans une enceinte de mélange de sorte que le premier rotor et le second rotor se font face l'un à l'autre et ont respectivement un arbre de rotation individuel sur la même ligne d'axe dans une direction approximativement horizontale, et qui mélange les matières premières par la rotation du premier rotor et du second rotor dans une direction identique ou opposée l'un par rapport à l'autre, tout en fournissant les matières premières à travers une ouverture d'alimentation sur le côté du premier rotor, et rejette les matières premières mélangées à travers une ouverture de rejet sur le côté du second rotor.

9. Procédé pour fabriquer un produit cosmétique sous forme de poudre solide selon la revendication 8, dans lequel le premier rotor et le second rotor de l'appareil de mélange de type à rotors opposés tournent dans une direction opposée l'un par rapport à l'autre.

10. Procédé pour fabriquer un produit cosmétique sous forme de poudre solide selon la revendication 8 ou 9, dans lequel une quantité du mélange d'amide est de 1,0 % à 15 % en masse.

11. Procédé pour fabriquer un produit cosmétique sous forme de poudre solide selon l'une quelconque des revendications 8 à 10, en mélangeant avec 1 % à 20 % en masse de particules sphériques de poly(méth)acrylate en tant que composant sous forme de poudre.

12. Procédé pour fabriquer un produit cosmétique sous forme de poudre solide selon la revendication 11, dans lequel les particules sphériques de poly(méth)acrylate sont des particules ayant une taille moyenne de particule de 3 à 20 µm, une surface spécifique de 80 à 180 m²/g et un diamètre de pore le plus fréquent de 180 Å ou plus.

13. Procédé pour fabriquer un produit cosmétique sous forme de poudre solide selon l'une quelconque des revendications 8 à 12, en mélangeant en outre avec un minéral argileux modifié organiquement en une quantité de 1,0 % à 15 % en masse en tant que quantité totale avec le mélange d'amide.

14. Procédé pour fabriquer un produit cosmétique sous forme de poudre solide selon l'une quelconque des revendications 8 à 13, en mélangeant avec 5 % à 97 % en masse de poudre traitée par un composé fluoré en tant que composant sous forme de poudre.

15. Procédé pour fabriquer un produit cosmétique sous forme de poudre solide selon la revendication 14, dans lequel le composé fluoré est le 1H,1H,2H,2H-perfluorooctyltriéthoxysilane.

16. Procédé pour fabriquer un produit cosmétique sous forme de poudre solide, comprenant :
une étape de préparation d'une suspension consistant à mélanger un composant sous forme de poudre, un composant huileux en tant que liant, et un mélange d'amide obtenu en amidant un mélange d'hexaméthylènediamine et de bisaminométhylcyclohexane avec des acides gras d'huile de ricin hydrogénée, dans un solvant volatil pour obtenir une suspension ; et
une étape de séchage consistant à sécher la suspension pour obtenir une poudre sèche, et
la production d'un produit cosmétique sous forme de poudre solide à partir de la poudre sèche obtenue,
dans lequel l'étape de séchage est mise en oeuvre en utilisant un appareil de séchage qui effectue le séchage de la suspension en convertissant la suspension en gouttelettes liquides fines au moyen d'une force de cisaillement mécanique et en soufflant un gaz sec sur les gouttelettes liquides fines.

17. Procédé pour fabriquer un produit cosmétique sous forme de poudre solide selon la revendication 16, dans lequel l'appareil de séchage est un appareil ayant un logement de forme creuse ; un dispositif de cisaillement qui cisaille une suspension au moyen d'éléments de cisaillement disposés à l'intérieur du logement et qui convertit la suspension en gouttelettes liquides fines ; un dispositif d'alimentation qui fournit la suspension aux éléments de cisaillement dans le logement ; un dispositif de soufflage de gaz qui souffle un gaz sec dans le logement, et apporte le gaz sec à la suspension qui a été convertie en gouttelettes liquides fines par le dispositif de cisaillement pour mettre le gaz sec et les gouttelettes liquides fines en contact ; et un dispositif de capture qui capture une poudre sèche produite par le séchage de la suspension.

18. Procédé pour fabriquer un produit cosmétique sous forme de poudre solide selon la revendication 16 ou 17, dans lequel, dans l'étape de préparation de suspension, une suspension est obtenue en mélangeant le composant sous forme de poudre et le composant huileux dans un solvant volatil en utilisant un broyeur agitateur de milieu, et le composant sous forme de poudre est craqué et/ou pulvérisé et/ou dispersé.

19. Procédé pour fabriquer un produit cosmétique sous forme de poudre solide selon l'une quelconque des revendications 16 à 18, dans lequel une quantité du mélange d'amide est de 1,0 % à 15 % en masse.

20. Procédé pour fabriquer un produit cosmétique sous forme de poudre solide selon l'une quelconque des revendications 16 à 19, en mélangeant avec 1 % à 20 % en masse de particules sphériques de poly(méth)acrylate en tant que composant sous forme de poudre.

21. Procédé pour fabriquer un produit cosmétique sous forme de poudre solide selon la revendication 20, dans lequel les particules sphériques de poly(méth)acrylate sont des particules ayant une taille moyenne de particule de 3 à 20 µm, une surface spécifique de 80 à 180 m²/g et un diamètre de pore le plus fréquent de 180 Å ou plus.

22. Procédé pour fabriquer un produit cosmétique sous forme de poudre solide selon l'une quelconque des revendications 16 à 21, en mélangeant en outre avec un minéral argileux modifié organiquement en une quantité de 1,0 % à 15 % en masse en tant que quantité totale avec le mélange d'amide.

23. Procédé pour fabriquer un produit cosmétique sous forme de poudre solide selon l'une quelconque des revendications 16 à 22, en mélangeant avec 5 % à 97 % en masse de poudre traitée par un composé fluoré en tant que composant sous forme de poudre.

24. Procédé pour fabriquer un produit cosmétique sous forme de. poudre solide selon l'une quelconque des revendications 16 à 22, dans lequel, dans l'étape de préparation de suspension, on mélange avec 0,1 % à 10 % en masse d'un composé fluoré.

25. Procédé pour fabriquer un produit cosmétique sous forme de poudre solide selon la revendication 23 ou 24, dans lequel le composé fluoré est le 1H,1H,2H,2H-perfluorooctyltriéthoxysilane.
